# EUROPEAN PATENT SPECIFICATION

(11) **EP 1 326 961 B1**
(45) Date of publication and mention of the grant of the patent: **22.08.2007**
(21) Application number: 01968626.0
(22) Date of filing: 06.09.2001
(51) Int. Cl.: C12N 5/00, C12N 5/02, A61K 39/00, A61K 39/38

(54) **COMPOSITIONS AND METHODS FOR INDUCING SPECIFIC CYTOLYTIC T CELL RESPONSES**
ZUSAMMENSETZUNGEN UND VERFAHREN ZUR AUSLÖSUNG SPEZIFISCHER ZYTOLYTISCHER T-ZELLANTWORTEN
COMPOSITIONS ET METHODES DESTINEES A INDUIRE DES REPONSES CYTOLYTIQUES SPECIFIQUES CHEZ DES LYMPHOCYTES T

(30) Priority: 15.09.2000 US 233009 P
(43) Date of publication of application: 16.07.2003
(73) Proprietor: Ortho-McNeil Pharmaceutical, Inc., Raritan, NJ 08869 (US)
(72) Inventor: VITIELLO, Antonella, La Jolla, CA 92037 (US); MACCARIO, Rita, I-27100 Pavia (IT); MONTAGNA, Daniela, I-27042 Bressana (IT)
(74) Representative: Mercer, Christopher Paul
(86) International application number: PCT/US2001/028016
(87) International publication number: WO 2002/022648

(56) References cited:
- WO-A-94/02156
- WO-A-99/42564
- DATABASE BIOSIS [Online] BIOSCIENCES INFORMATION SERVICE, PHILADELPHIA, PA, US; 16 November 2000 (2000-11-16), MONTAGNA DANIELA ET AL: "Dendritic cells pulsed with leukemia blasts elicit in vitro potent anti-leukemia cytotoxic T-cell response in patients given allogeneic bone marrow transplantation and in their donors" XP002330526 Database accession no. PREV200100293826 & BLOOD, vol. 96, no. 11 Part 1, 16 November 2000 (2000-11-16), page 175a, 42ND ANNUAL MEETING OF THE AMERICAN SOCIETY OF HEMATOLOGY; SAN FRANCISCO, CALIFORNIA, USA; DECEMBER 01-05, 2000 ISSN: 0006-4971
- BHARDWAJ N ET AL: "IL-12 in conjunction with dendritic cells enhances antiviral CD8+ CTL responses in vitro" JOURNAL OF CLINICAL INVESTIGATION, NEW YORK, NY, US, vol. 98, no. 3, August 1996 (1996-08), pages 715-722, XP002127215 ISSN: 0021-9738
- CHIKAMATSU K ET AL: "Generation of anti-p53 cytotoxic T lymphocytes from human peripheral blood using autologous dendritic cells" CLINICAL CANCER RESEARCH, THE AMERICAN ASSOCIATION FOR CANCER RESEARCH, US, vol. 5, no. 6, June 1999 (1999-06), pages 1281-1288, XP002306894 ISSN: 1078-0432
- MONTAGNA DANIELA ET AL: "Ex vivo priming for long-term maintenance of antileukemia human cytotoxic T cells suggests a general procedure for adoptive immunotherapy" BLOOD, vol. 98, no. 12, 1 December 2001 (2001-12-01), pages 3359-3366, XP002330525 ISSN: 0006-4971
- BAXEVANIS C.N. ET AL.: 'Tumor specific CD4+ T lymphocytes from cancer patients are required for optimal induction of cytotoxic T cells against the autologous tumor' THE JOURNAL OF IMMUNOLOGY vol. 164, 2000, pages 3902 - 3912, XP002908498
- GALLUCCI S. ET AL.: 'Natural adjuvants: Endogenous activators of dendritic cells' NAT. MED. vol. 5, no. 11, November 1999, pages 1249 - 1255, XP002908497
- FALKENBURG J.H.F. ET AL.: 'Complete remission of accelerated phase chronic myeloid leukemia by treatment with leukemia-reactive cytotoxic T lymphocytes' BLOOD vol. 94, no. 4, 15 August 1999, pages 1201 - 1208, XP002908496
- ALBERT M.L. ET AL.: 'Immature dendritic cells phagocytose apoptotic cells via alphaVbeta5 and CD36 and cross-present antigens to cytotoxic T lymphocytes' J. EXP. MED. vol. 188, no. 7, 05 October 1998, pages 1359 - 1368, XP000906793
- ALBERT M.L. ET AL.: 'Dendritic cells acquire antigen from apoptotic cells and induce glass I-restricted CTLs' NATURE vol. 392, 05 March 1998, pages 86 - 89, XP002154749
- CARDOSO A.A. ET AL.: 'Ex vivo generation of human anti-Pre-B leukemia-specific autologous cytolytic T cells' BLOOD vol. 90, no. 2, 15 July 1997, pages 549 - 561, XP002908495

## Description

### BACKGROUND OF THE INVENTION

This invention relates generally to diseases and conditions that are mediated by pathologically aberrant cells, such as autoimmune disorders, infectious diseases, allergy, and proliferative diseases such as cancer and, more specifically, to methods of preparing immune cells that can be used to treat cancer and other cell proliferation disorders.

Cancer is one of the leading causes of death in the United States. Each year, more than half a million Americans die from cancer, and more than one million are newly diagnosed with the disease. Cancerous tumors result when a cell escapes from its normal growth regulatory mechanisms and proliferates in an uncontrolled fashion. Tumor cells can metastasize to secondary sites if treatment of the primary tumor is either not complete or not initiated before substantial progression of the disease. Cancer-related mortality can be decreased by prevention, early detection, and rigorous therapies.

A hurdle to treating cancer is the relative lack of agents that can selectively target the cancer, while sparing normal tissue. For example, radiation therapy and surgery, which generally are localized treatments, can cause substantial damage to normal tissue in the treatment field, resulting in scarring and in severe cases, loss of function of normal tissue. Chemotherapy, which generally is administered systemically, can cause substantial damage to organs such as bone marrow, mucosae, skin and the small intestine. Other cancer therapies include antibodies directed to tumor cell surface proteins, and antibodies conjugated to cytotoxic agents. Therapeutic antibodies are targeted to tumor cells by the recognition of tumor-specific cell surface proteins. The production of antibodies that bind or deliver toxin to a particular tumor therefore requires that a protein target be identified, and that the protein be exposed on the cell surface. The efficacy and side effects of antibody therapies vary, and some therapeutic antibodies can cause serious allergic responses in individuals.

Other therapies involving the use of the body's immune system to fight cancer are under development. One immunotherapy approach aims to stimulate the immune system of the cancer patient indirectly using agents such as vaccines and cytokines. A more direct immunotherapy approach is to increase the anti-tumor immunity of the patient by administering immune cells that attack the tumor. The latter method, referred to as adoptive immunotherapy, has been used with varying degrees of success to treat patients suffering from several cancers, including malignancies of the brain, kidney, skin, and blood. Long-term cancer regression after adoptive immunotherapy treatment has not been reproducibly observed.

One method of adoptive immunotherapy involves using tumor-specific cytotoxic T lymphocytes (CTLs). CTLs are a specialized variety of CD8⁺ T lymphocytes that are capable of recognizing an antigen on the surface of a cell in association with Class I molecules of the Major Histocompatibility Complex (MHC) and subsequently destroying the cell. The role of CTLs in the immune system is to recognize and eliminate infected cells, tumor cells, and foreign cells. For adoptive immunotherapy, a population of anti-tumor CTLs derived from the patient, or a donor, can be generated using ex vivo culture methods.

Methods for generating anti-tumor CTLs ex vivo using peptides derived from tumor associated antigens and subsequently administering the CTLs to a cancer patient have been reported with varying success. However, such approaches for generating CTLs are limited to cases in which a tumor-specific antigen is known and appropriately processed to generate peptides that are presented by antigen presenting cells. Another drawback to ex vivo peptide induced CTL is the unpredictability of whether the resulting CTL will retain the ability to recognize the corresponding antigen on the tumor cell surface because the affinity of the CTLs generated against the peptide might not be sufficient to recognize the endogenously processed antigen. Therefore, the desired specificity or efficacy needed for therapeutic use of ex vivo generated CTL has been generally difficult to achieve. Another problem with ex-vivo generated CTL has been the difficulty in maintaining the CTL in vitro for more than 2 or 3 rounds of restimulation. Moreover, this method is further limited by generating CTLs that recognize only one antigen on the target tumor cell.

Thus, there exists a need to generate and maintain selective CTLs in culture for adoptive immunotherapy. The present invention satisfies this need and provides related advantages as well.

### SUMMARY OF THE INVENTION

The invention provides An *ex vivo* method of inducing CD8⁺ T lymphocytes (TL) selective for a pathologically aberrant cell, comprising:
(a) contacting said pathologically aberrant cell with a first mixture having at least dendritic cells (DC) and isolated CD4⁺ T cells for sufficient time to
(b) produce DCs presenting pathologically aberrant cell antigen; adding CD8⁺ TL to produce a second mixture;
(c) culturing said second mixture for sufficient time to generate CD8⁺ TL
(d) having antigenic specificity for said pathologically aberrant cell; culturing said CD8⁺ TL having antigenic specificity for said
(e) pathologically aberrant cell for two or more generations; and isolating CD8⁺ memory TL, said CD8⁺ memory TL being characterized as having the ability to produce CD8⁺ TL having antigenic specificity for
said pathologically aberrant cell.

The invention further provides An *ex vivo* method of inducing CD8⁺ T cells selective for a pathologically aberrant cell, comprising: contacting an apoptotic pathologically aberrant cell with a mixture having at least dendritic cells (DC), CD4⁺ T cells and CD8⁺ T lymphocytes; culturing said apoptotic pathologically aberrant cell with said mixture for sufficient time to generate CD8⁺ T lymphocytes (TL) having antigenic specificity for said pathologically aberrant cell; wherein IL-7 is added to the mixture or to the culture of apoptic pathologically aberrant cell and the mixture; culturing said CD8⁺ TL having antigenic specificity for said pathologically aberrant cell for two or more generations; and isolating CD8⁺ memory TL, said CD8⁺ memory TL being characterized as having the ability to produce CD8⁺ TL having antigenic specificity for and cytolytic activity against said pathologically aberrant cell;
wherein IL-7 is added in at least one of step (a), step (b), or step (c).

### BRIEF DESCRIPTION OF THE DRAWINGS

**FIGURE 1- Panel A, Panel B, Panel C and Panel D:**
   Shows cytotoxic activity of CD8 cells induced by immature dendritic cells and dendritic cells treated with LPS, TNF, or CD40L, and the cytotoxic activity of CD8 cells induced by immature or CD40L-treated dendritic cells in the presence of IL-12, IL-7, or IL-12 and IL-7.
**FIGURE 2 - Panel A, Panel B, Panel C and Panel D:**
   Shows cytolytic activities of LB-specific CTL lines derived from PBMCs of bone marrow transplant recipients, bone marrow cells of bone marrow transplant donors and from PBMCs of bone marrow transplant donors. Four donor/recipient pairs are represented in panels A-D.
**FIGURE 3:**
   Shows that CTL numbers increase after each in vitro stimulation with LBs.

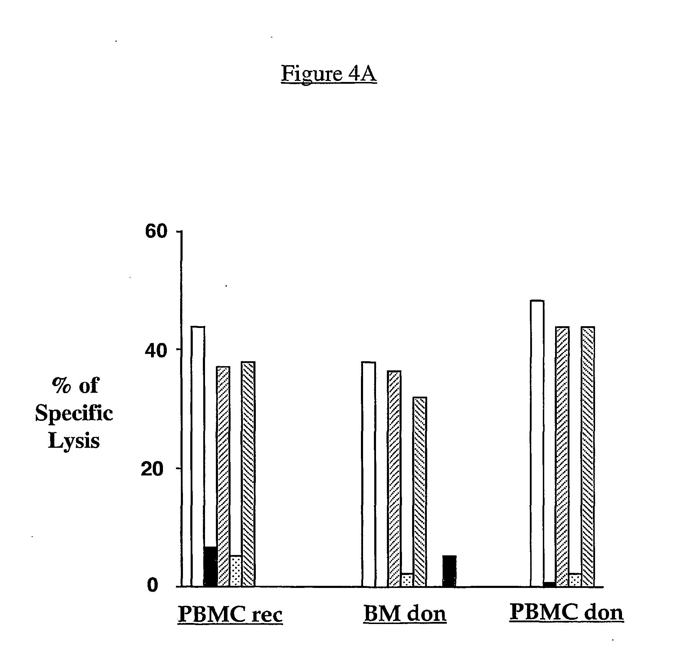

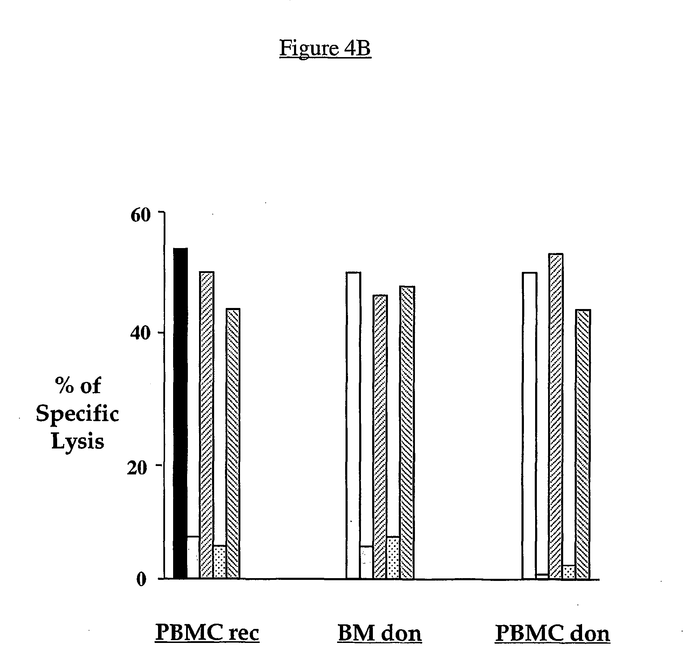

**FIGURE 4 - Panel A and Panel B:**
   Shows that LB-specific CTL lines are CD8⁺, class I restricted.

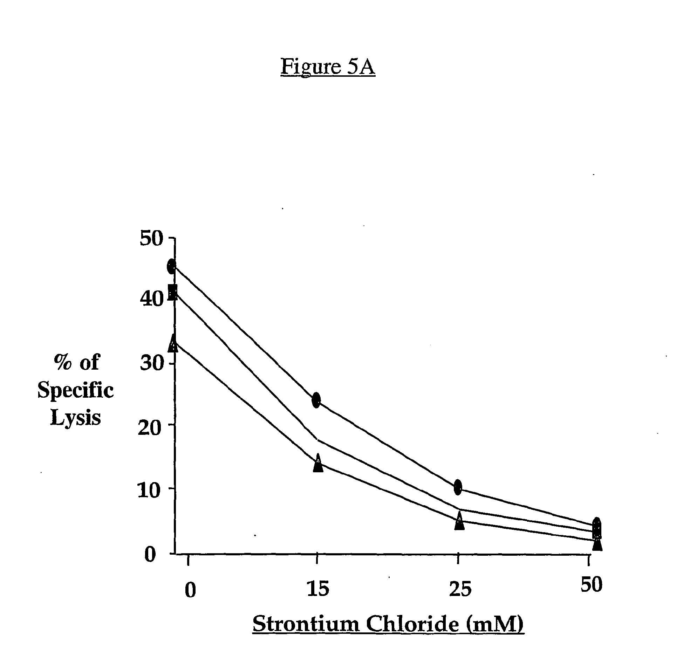

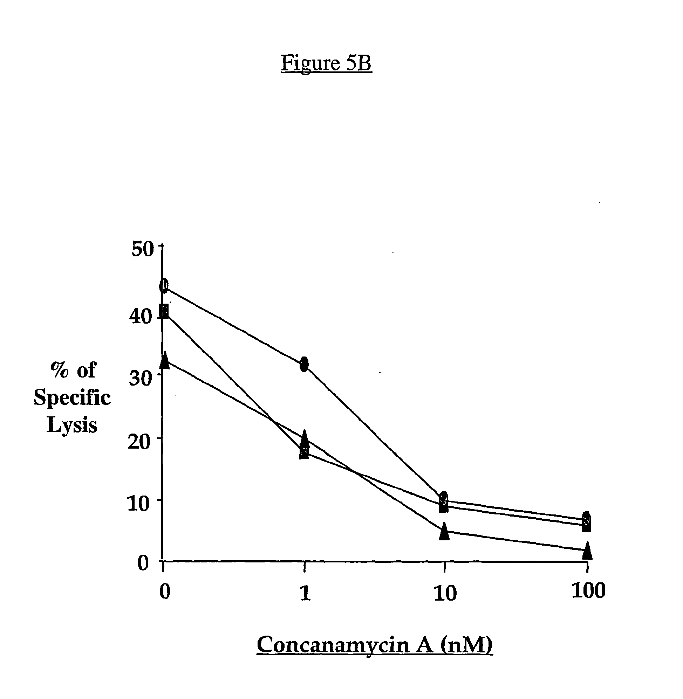

**FIGURE 5 - Panel A and Panel B:**
   Shows that CTL-mediated LB lysis is perforin dependent.

### DETAILED DESCRIPTION OF THE INVENTION

This invention is directed to methods of generating CD8⁺ T cells for use in treating an individual having a disease mediated by a pathologically aberrant cell. Provided are methods as defined above for ex vivo production of CD8⁺ T lymphocytes (TL) that have antigenic specificity for, and/or cytolytic activity selective for a pathologically aberrant cell, such as a tumor cell, a cell lysate, a cell fraction, a cell component, or a vesicle, or a B cell, a cell producing a substance that mediates a disease or a condition, or cell rendered abnormal due to the effects of an infectious agent. In addition, a cell can be considered abnormal because it produces abnormal proteins or proteins that albeit normal under certain circumstances, are involved with the induction or progression of disease. The CD8⁺ T lymphocytes having cytolytic activity against a target cell, such as a pathologically aberrant cell, are referred to as a CTL. An advantage of this method is that CTLs selective for a whole pathologically aberrant cell can be generated, eliminating the need to identify a specific tumor or disease-related antigen. This method also eliminates unknown factors associated with processing and presentation of peptide antigens. Another advantage of the method is that CTLs generated against a whole target cell can provide a poly-specific response against a pathological target. Furthermore, induction of CTLs using the whole cell allows for the selection of the CTLs that are present in each individual's CD8⁺ T cell repertoire. In fact, it is known that, in some patients, some CTL specificities may be absent due to tolerance. Moreover, generating CTLs against the whole tumor cell allows for the induction of CTLs specific for individual mutations that have occurred in the target cell.

The invention also provides methods for generating CD8⁺ CTLs, and their precursor antigen-specific CD8⁺ TL, that can be maintained in vitro for longer periods of time, which is a characteristic of CD8⁺ memory cells. CD8⁺ memory cells are progenitors for the expansion of CTLs that, when administered therapeutically, can provide a patient with long-term immunity against a selectively targeted pathologically aberrant cell. CD8+ memory cells refer to a CD8+ cell that has the capacity to proliferate in vitro for at least five rounds of stimulation, maintaining its antigen specificity. The stimulation can be maintained by the antigen or by non-specific means including, for example by mitogens or antibodies directed against the T cell receptor or co-stimulatory molecules or by procedures well known to those skilled in the art.

Selective CTLs and antigen-specific CD8⁺ TL, that recognize pathologically aberrant cells are useful for adoptive immunotherapy approaches to treating cancer, as well as other diseases in which the elimination of abnormal or pathologically aberrant cells can provide effective treatment. CTLs that selectively destroy a pathologically aberrant cell are useful for treating disease because the elimination of diseased cells can reduce symptoms associated with the presence or unwanted proliferation of pathologically aberrant cells. The generation of CTLs selective for pathologically aberrant cells for use in adoptive immunotherapy will increase the treatment options available for reducing the symptoms of, or curing, conditions in which destruction or reduction of pathologically aberrant cells would provide benefit.

Herein described is the ex vivo generation of CTLs with selective cytolytic activity toward tumor cells. Tumor-selective CTLs are prepared by incubating CD8⁺ T cells and dendritic cells from the peripheral blood of a donor with apoptotic tumor cells isolated from an individual having acute myeloid leukemia (AML) who will receive the product CTLs in an adoptive immunotherapy treatment. Dendritic cells and apoptotic pathologically aberrant cells are incubated together for sufficient time so that dendritic cells present antigens of the pathologically aberrant cell. Isolated CD8⁺ TL from the donor are added to the mixture of dendritic cells and apoptotic pathologically aberrant cells in the presence of IL-7 and IL-12, and the populations are incubated for a sufficient time to produce antigen-specific CD8⁺ TL and selective CTLs. The resulting tumor-selective CTLs can be administered to the bone marrow recipient to treat or reduce the severity of disease.

In an embodiment, the invention is directed to the ex vivo generation of CD8⁺ memory T cells from which a population of CTLs selective for a tumor cell can be expanded. CD8⁺ memory T cell populations prepared using the method of the invention can reproducibly survive repeated in vitro stimulation with antigen. The method involves culturing a mixture of dendritic cells, CD8⁺ TL and CD4⁺ T cells with target tumor cells in the presence of IL-7. The resulting CD8⁺ cell population contains antigen-specific CD8⁺ TL, CTLs selective to the tumor cell and, after two or more generations, contains CD8⁺ memory TL that can be expanded ex vivo for five or more generations.

Anti-tumor CTL generated using the methods of the invention can be utilized to treat an individual having cancer. For example, adoptive immunotherapy can be used to introduce anti-tumor immunity to a patient with a hematopoietic cancer who has cancer relapse after receiving a bone marrow transplant. Anti-tumor CTL can be generated from the CD8⁺ TL, dendritic cells, and. CD4+ T cells of the bone marrow donor combined with the tumor cells from the patient. In this case, in which tumor cells of the BMT recipient are HLA identical to the CD8⁺ TL and CD4⁺ T cells and dendritic cells of the bone marrow donor, naive donor CD8⁺ TL exhibit a primary immune response against non-major HLA antigens. Using the methods of the invention, the generation of CD8⁺ memory TL allows for long-term maintenance of these ex vivo induced primary responses.

For example, anti-tumor CTLs generated using the methods of the invention were found to be induced from the peripheral blood and bone marrow of the bone marrow donor, as well as from the peripheral blood of the bone marrow recipient, six months after the adoptive immunotherapy procedure. The generation of tumor-selective CTLs that have immunological memory will improve the probability that long-term protective immunity can be provided by adoptive therapy treatment.

As used herein, the term "pathologically aberrant cell" refers to a cell that is altered from normal due to changes in physiology or phenotype associated with a disease or abnormal condition of a mammalian cell or tissue. A pathologically aberrant cell can be distinguished from a normal cell due to an alteration that has occurred that causes a disease or abnormal condition, or that is the result of a disease or abnormal condition. From these pathologically aberrant cells can be made sub-cellular preparations, such as cell lysates, cell fractions, cell components or vesicles, using standard techniques known in the art, such as chemical and physical disruption, centrifugation, gradient separation and chromatography. Such sub-cellular preparations are useful in the methods of the present invention and can be substituted for whole cell preparations of pathologically aberrant cells.

Pathologically aberrant cells include cells that have a loss of regulation or control of a cellular function. Loss of control of a cellular function can lead to cellular changes that distinguish a pathologically aberrant cell from normal. Examples of cellular functions are proliferation and differentiation. Loss of control of proliferation can result in cellular changes that cause an abnormal condition of a cell or tissue. For example, cancer cells are abnormal and proliferate in an unregulated manner, which results in tissue destruction. As used herein, the term "tumor cell" refers to a cancer cell. Specific examples of cancers include prostate, breast, lung, ovary, uterus, brain and skin cancer. Similarly, the proliferation of cells mediating autoimmune diseases are aberrantly regulated, which results in, for example, the continued proliferation and activation of immune mechanisms with destruction of the host's cells and tissue. Autoimmune diseases include, for example, rheumatoid arthritis, diabetes, and multiple sclerosis.

Pathologically aberrant cells include, for example, cells that produce substances capable of mediating a disease or condition. An example of such a pathologically aberrant cell is a B cell that produces an IgE antibody responsible for the appearance of allergic symptoms. Pathologically aberrant cells also include cells and specific cell types found in specific organs of the body, whether the organ is a vital or non-vital organ. For example, an organ may be comprised of several different cell types, such as epithelial, myocyte and/or fibroblast. One or more of these cells types may be pathologically aberrant, and as such can be targeted by the CD8⁺ TL and CTL produced by the methods of the present invention.

Loss of regulation or control of cellular functions can also occur due to infection of a cell by a pathological agent. The term "pathological agent" refers to an infectious agent that causes disease. Examples of pathological agents include viruses, bacteria, fungi, amoeba, and parasites. Specific examples of infectious diseases include DNA or RNA viral diseases, bacterial diseases, and parasitic diseases.

The term "apoptosis" refers to the process of programmed cell death. Programmed cell death is a regulated process in which a cell responds to a specific physiological or developmental signal and undergoes a programmed series of events that leads to its death and removal from the organism. Examples of the cellular events that characterize apoptosis are cell shrinkage, mitochondrial break down with the release of cytochrome c, cell surface blebbing, chromatin degradation, and phosphatidylserine exposure on the surface of the plasma membrane. Apoptosis is distinct from necrosis, or cell death that results from injury, which is characterized by overall cell and organelle swelling, with subsequent early loss of membrane integrity followed by cell and organelle lysis. Necrosis, unlike apoptosis, is accompanied by an inflammatory response in vivo.

The term "CD4⁺ T cells" refers to lymphocytes that produce the CD4 protein, and are able to interact with dendritic cells to induce antigen presentation by or maturation of the dendritic cells. Such CD4⁺ T cells include, but are not limited to, cells isolated from natural sources such as blood, cell lines grown in culture, and CD4⁺ T cell clones.

The term "selective", when used in reference to CD8⁺ cytolytic T cell, is intended to mean a CD8⁺ cytolytic T lymphocyte (CTL) that preferentially recognizes and has cytolytic activity toward a particular pathologically aberrant target cell, compared to a non-target cell. A selective CTL can distinguish, or can be made to distinguish, a target pathologically aberrant cell from a population of non-target cells, and does not substantially cross-react with non-target cells. When used in reference to immune reactivity or antigenic specificity, the term "selective" is intended to mean that a T cell receptor of a CTL or CD8⁺ TL preferentially binds to a particular target antigen. A CTL that exhibits selective immune reactivity does not substantially cross-react with non-target antigens, and can distinguish a pathologically aberrant cell exhibiting the target antigen from a normal or non-target cell.

The term "ex vivo" when used in reference to a cell is intended to mean a cell outside of the body. Therefore, an ex vivo cell culture method involves harvesting cells from an individual. Ex vivo culture methods are applicable to a cell harvested from any tissue or organ of an individual. Cell culture conditions of ex vivo cultures include a variety of compositions. Cells can be in a heterogeneous mixture or can be isolated cells. Medium can be an undefined or defined cell culture medium or can contain added factors. Medium can contain factors that enhance the therapeutic potential of the cells. For example, factors can be used to promote growth, viability or differentiation. Added factors can include other cells, protein factors, and chemical reagents.

The term "sufficient time" is intended to mean a period of time that allows a CD8⁺ T cell to be induced. The CD8⁺ T cell induction process includes at least processing and presenting of an antigen by dendritic cells, recognition by CD8⁺ T cells of an antigen, and activation of cytolytic activity. A sufficient time that allows for the completion of this process can be a small or large period of time because differences in the various cell populations of the methods will result in differences in rates of antigen uptake. Factors that can affect the sufficient time for CD8⁺ T cell induction can be, for example, the types of cells in a culture, the purity of various cell types, concentrations of cell types, and whether dendritic cells are immature or are presenting antigen at the time of culture. A sufficient time can occur instantaneously, within minutes, hours, days or weeks, depending on the composition of the reaction mixture. For example, when a CD8⁺ T cell is added to a reaction mixture that contains prepared mature dendritic cells presenting antigen, priming of the CD8⁺ T cells will occur in a relatively small period of time because the step of dendritic cell antigen uptake and processing has already occurred compared to a case in which a CD8⁺ T cell is added to reaction mixture that contains immature dendritic cells and target pathologically aberrant cells or other antigen(s).

As used herein, term "isolated" refers to a cell that is separated from one or more components with which it is associated in nature. An isolated cell also includes a cell purified from non-cellular tissue components, such as connective tissue fibers. An isolated cell can be, for example, a primary cell, either freshly purified from non-cellular tissue components, or cultured for one or more generation. An example of an isolated cell is a cell that that has been separated from blood, such as a cell of a preparation of peripheral blood mononuclear cells (PBMCs).

The term "substantially" when used in reference to an isolated cell is intended to mean a cell represents 90-99% of a purified cell population. For example, a substantially isolated cell can be 90-95% pure, 95 % pure, 95-99% pure, or can be as pure at 99% or greater.

The term "substantially purified" when used in reference to CD40 Ligand (CD40L) or IL-12 is intended to mean that the proteins are substantially free of other components with which they are naturally associated. For example, CD40L and IL-12 proteins are normally found with other proteins in cells. These proteins may act as unwanted contaminants when CD40L or IL-12 is used to treat cultured cells.

The term "non-B-cell leukemia cell" refers to any cell type that is not a B cell-leukemia cell or a pre-B cell leukemia cell. A non-B-cell leukemia cell includes a normal non-cancerous B cell, and also includes a B cell of a non-leukemia cancer, such as a B-cell lymphoma cell. Non-B-cell leukemia cells include other types of leukemia cells, such as T-cell leukemia cells. Non-B-cell leukemia cells can be any type of cancer cell that is not a B-cell or pre-B-cell leukemia cell, and can be non-cancer cells of any type, including cells that are pathologically aberrant due to a non-cancer condition or disease.

As used herein, term "antigen" is intended to mean a molecule that can be processed and presented by an antigen-presenting cell and subsequently recognized by a T cell receptor. Such a molecule can be for example, a polypeptide.

The term "target", when used in reference to the immune reactivity of a CD8⁺ T cell is any predetermined antigen. A predetermined antigen can be, for example, a cell or polypeptide.

As used herein, the term "naive" when used in reference to a CD8⁺ T cell is intended to mean that a CD8⁺ T cell, has either not seen a particular target cell or antigen in vivo. Therefore, a naive CD8⁺ T cell must be exposed to a particular target cell or antigen ex vivo in order for it to be capable of CTL activity selective for the particular target cell or antigen.

As used herein, the term "in situ" when used in reference to selective CTL activity is intended to mean that selective CTLs can destroy a target pathologically aberrant cell in an intact structure of the body. For example, a selective CTL can destroy a target cell in a heterogeneous population of cells. Specifically, a selective CTL can eliminate a pathologically aberrant cell, such as a tumor cell, from a tissue, such as blood or bone marrow.

As used herein, the term "treating" is intended to mean reduction in severity or prevention of a pathological condition mediated by a pathologically aberrant cell. Reduction in severity includes, for example, an arrest or decrease in clinical symptoms, physiological indicators, biochemical markers or metabolic indicators. Prevention of disease includes, for example, precluding the occurrence of the disease or restoring a diseased individual to their state of health prior to disease.

As used herein, the term "effective amount" is intended to mean an amount of CD8⁺ cytolytic T cells required to effect a decrease in the extent, amount or rate of spread of a pathological condition when administered to an individual. The dosage of a CTL preparation required to be therapeutically effective will depend, for example, on the pathological condition to be treated and the level of abundance and density of the target antigens as well as the weight and condition of the individual, and previous or concurrent therapies. The appropriate amount considered as an effective dose for a particular application of selective CTLs provided by the method can be determined by those skilled in the art, using the guidance provided herein. For example, the amount for administration can be extrapolated from in vitro or in vivo cytotoxicity assays as described below. One skilled in the art will recognize that the condition of the patient needs to be monitored throughout the course of therapy and that the amount of the composition that is administered can be adjusted according to the individual's response to therapy.

The invention provides a method of inducing the cytolytic activity of CD8⁺ T cells specific for a pathologically aberrant cell ex vivo. The method consists of contacting an apoptotic pathologically aberrant cell with a mixture having at least dendritic cells (DC), CD4⁺ T cells and CD8⁺ T cells, and culturing said apoptotic pathologically aberrant cell with said mixture for sufficient time to generate CD8⁺ cytolytic T cells (CTL) having selective cytolytic activity toward said pathologically aberrant cell.

The invention further provides an ex vivo method of inducing CD8⁺ T cells specific for a pathologically aberrant cell that is a non-B-cell leukemia cell.

The methods of the invention can be used to generate a population of CTLs selective for any apoptotic pathologically aberrant cell or non-B-cell leukemia pathologically aberrant cell, preferably when elimination of a pathologically aberrant cell can provide benefit to an individual. Such benefits can include reducing disease severity, disease symptoms, disease progression, or rate of disease progression. Particular types of pathologically aberrant cells include, for example, those cells that are abnormally regulated compared to a normal cell. Pathologically aberrant cells can be cells that are changed from normal due to disease, or can be altered cells that cause disease by their presence, or cells that produce factors that cause disease. Therefore, the methods of the invention are applicable to pathologically aberrant cells that mediate diseases such as cancer, autoimmune disorders, infectious diseases, and allergies.

By specific mention of the above categories of pathological conditions, those skilled in the art will understand that such terms include all classes and types of these pathological conditions. For example, the term cancer is intended to include all known cancers, whether characterized as malignant, benign, soft tissue or solid tumor. By exemplification, a non-exhaustive list of known cancers is provided below in Table 1. Similarly, and by analogy to the classes and types of cancers shown in Table 1, the terms infectious diseases and autoimmune diseases are intended to include all classes and types of these pathological conditions. Those skilled in the art are familiar with the various classes and types of infectious and autoimmune diseases and allergies.

**Table 1.**

| **TUMOR AND CANCER TYPES** |
|---|
| adrenal tumor, acoustic neuroma, adenocarcinoma, acral lentiginous melanoma, arrhenoblastoma, atrial myxoma, astrocytoma |
| basal cell cancer, benign ear tumors, bile duct cancer, bone neoplasm, bone tumor, brain tumor (primary, secondary and metastatic), breast cancer, bronchogenic carcinoma, Burkitt's tumor |
| cancer of the cervix, cancer of the esophagus, cancer of the kidney or ureter, cancer of the penis, cancer of the perineum, cancer of the testicle, cancer of the vulva, carcinoma of the renal pelvis or ureter, carcinoma of the stomach, carcinoma of the testes, cerebellopontine angle tumor, colon cancer, colorectal cancer, choriocarcinoma, chorioblastoma, chorioepithelioma, craniopharyngioma, cancer of the uterus, cancer of the larynx or vocal chords. |
| endometrial cancer, ependymoma, Ewing's sarcoma |
| gastric cancer, glioblastoma multiforme |
| hepatocellular carcinoma, Hodgkin's lymphoma, histiocytic lymphoma, hypernephroma, heart tumor |
| intestinal cancer, islet cell tumors |
| large bowel neoplasm, leukemia, laryngeal cancer, liver cancer, lung cancer, lymphocytic lymphoma, lymphoblastic lymphoma, lentigo maligna melanoma |
| malignant melanoma, malignant plasmacytoma, meduloblastoma, meningioma, metastatic cancer to the lung, metastatic pleural tumor, multiple myeloma, myxoma |
| nephroblastoma, neuroglioma, non-Hodgkin's lymphoma, non-small cell lung cancer |
| Oligodendroglioma, osteosarcoma, osteogenetic carcinoma, oral cancer |
| Pancreatic cancer, pituitary tumor, plasma cell myeloma, prostatic neoplasm |
| renal cell carcinoma, renal neoplasm, retinoblastoma, reticulum cell carcinoma |
| Schwannoma, salivary duct tumor, sarcoma, sarcoma botryoides, seminoma, Sertoli-Leydig cell tumor, small cell lung cancer, squamous cell cancer, spinal cord tumor |
| Throat cancer, thyroid-medullary carcinoma, thyroid cancer, trophoblastic tumor |
| Wilms tumor |

The methods of the invention are applicable to producing CTL cells that are selective for pathologically aberrant cells that are aberrantly regulated. Aberrantly regulated cells include, for example, cells that exhibit uncontrolled cell proliferation as well as cells that exhibit dysfunction in specific phases of the cell cycle, leading to altered proliferative characteristics or morphological phenotypes. Specific examples of aberrantly regulated cell types include neoplastic cells such as cancer and hyperplastic cells characteristic of tissue hyperplasia. Another specific example includes immune cells that become aberrantly activated or fail to down regulate following stimulation. Such aberrantly regulated immune cells mediate autoimmune diseases. Aberrantly regulated cells also includes cells that are biochemically or physiologically dysfunctional. Other types of aberrant regulation of cell function or proliferation are known to those skilled in the art and are similarly pathologically aberrant cells applicable to methods of generating CTLs of the invention.

Pathologically aberrant cells include aberrantly regulated cells such as those described above, and additionally include, for example, cells that are infected with a pathological agent, such as an infectious agent. Infectious agents that render a cell abnormal include, for example, those agents that require host cell machinery for survival or propagation. For example, viruses infect cells and cause cancer. Included within such infectious agents is DNA viruses, RNA viruses and parasites. Specific examples of DNA viruses include adenoviruses and parvoviruses. Specific examples of RNA viruses include poliomyelitis, influenza and retroviruses. Viruses that rapidly mutate are applicable to the methods of the invention. The use of a pathologically aberrant cell as a source of antigen can provide CTL cells that are specific for multiple epitopes on a cell infected with a rapidly mutating virus. Compared to a CTL population that selectively recognizes one target antigen, a CTL population selective for multiple target antigens can increase the probability that a virus-containing cell will be destroyed. Parasites that utilize eukaryotic host cell machinery for survival or propagation include, for example, trypanosomes. The cells infected by these and other agents known in the art, which utilize host cell machinery, are rendered abnormal because they are compromised in normal cellular function, which can manifest in morphological and biochemical changes. As such, CTLs generated by the methods of the invention can target these cells.

Pathologically aberrant cells include cells that are abnormal due to changes in proteins, such as changes in regulation of normal protein expression. Changes in protein expression include increased expression of a protein, expression of a foreign protein, expression of a protein not normally expressed in a particular cell type at a particular stage, and expression of a mutant protein. Pathologically aberrant cells having changed protein expression from normal can also have cell surface antigens that are distinct from those of normal cells. Pathologically aberrant cells also include cells that are making a protein that causes a disease or condition, such as B cells making IgE antibodies specific for an allergen, or other types of antibodies that can mediate other diseases or conditions.

Pathologically aberrant cells that are non-B-cell leukemia cells, including non-pre-B-cell leukemia cells, can be B cells that are pathologically aberrant due to a disease or condition other than leukemia. For example, a non-B-cell leukemia cell can be a different type of B-cell cancer cell, such as a B-cell lymphoma cell. Non-B-cell leukemia cells can be leukemia cells of a type other than B cell or pre-B-cell leukemia cells. For example, a non-B-cell leukemia cell can be a T-cell leukemia cell. A B-cell leukemia cell can be a B cell or pre-B cell that is not expressing CD40. Such a cell is incapable of responding to CD40L.

All of the above aberrant and abnormal cell classifications and cell types exhibit undesirable physiological characteristics and phenotypes that can lead to unwanted cell growth and complications. As such, these aberrantly regulated or abnormal cells will exhibit differences in antigen synthesis or expression compared to normal cells. These differences can be distinguished by CTL that are selective for the target pathologically aberrant cells generated by the methods of the invention.

According to the methods of the invention, a population of CD8⁺ T cells is induced to generate cytolytic T lymphocytes (CTLs) that are selective for a pathologically aberrant cell. A cell that is selectively recognized by a CTL will be destroyed by either direct cytotoxicity or by the induction of apoptosis. CTLs are mature CD8⁺ TL that recognize antigen in the form of polypeptide fragments complexed with MHC class I molecules. The binding of an antigen to a T cell receptor (TCR) complexed on the surface of a CD8⁺ TL is one event involved in the initiation of intracellular changes that lead to differentiation of CD8⁺ TL to cytolytic T lymphocytes (CTLs). CTLs exhibit cytolytic activity toward cells that display a target antigen which can be bound by a TCR.

In order to generate CTLs that are selective for a pathologically aberrant cell, CD8⁺ TL are primed by a pathologically aberrant cell in the presence of cells or factors that augment the CTL priming process. Therefore, priming is the exposure of CD8⁺ T cells to become active CTLs that selectively recognize and lyse pathologically aberrant cells expressing the inductive antigen. One requirement for induction of CTL is the presentation of antigen by an antigen-presenting cell. Nearly all nucleated cells express MHC class I, but may not be capable of priming CD8⁺ T cells because additional co-receptors, co-stimulatory molecules and other factors are necessary for efficient priming. Therefore, the use of a professional antigen presenting cell, which can express all of the required factors for priming, can increase the efficiency CTL priming in a mixture of cells cultured ex vivo for priming CTLs selective for a pathologically aberrant cell.

Dendritic cells are professional antigen-presenting cells (a term well known to and commonly used by those skilled in the art) that can efficiently process and present antigens and potently induce CD8⁺ TL cytolytic responses. Presentation of antigens by dendritic cells can occur after dendritic cell phagocytosis of an apoptotic cell. Therefore, in combination with a dendritic cell, a pathologically aberrant cell which is apoptotic, or which will undergo apoptosis, can be used as a target cell in the methods of the invention.

A cell that performs the function of a dendritic cell can be substituted for a dendritic cell in the methods of the invention. Such a substitute for a dendritic cell could be, for example, a cell that presents antigens or co-stimulatory molecules due to expression of a recombinant protein. Expression of recombinant proteins to produce such artificial antigen presenting cells is well known in the art and those of skill could determine a recombinant protein for expression in an artificial antigen-presenting cell for use in the methods of the invention.

The methods of the invention therefore utilize at least a CD8⁺ TL, a dendritic cell and a pathologically aberrant cell, which is apoptotic or which will undergo apoptosis, for the induction of CTLs selective for the target pathologically aberrant cell. Other cells and factors added to this mixture can augment the efficiency of priming or level of CTL response of the initial CD8⁺ T cells. For example, the inclusion of CD4⁺ T cells in the CTL induction mixture can provide additional factors required for efficient priming and production of a CD8⁺ memory CTL.

CD4⁺ T cells provide T cell help that enables CD8⁺ TL response to cellular antigens. In vivo, this T cell help results from the activation of dendritic cells by CD4⁺ T cells and can be mediated by CD40-CD40L interaction. Several factors can substitute for a function provided by a CD4⁺ T cell in an incubation of a mixture of cells for priming a CD8⁺ T cell.

For example, it is herein described that CD40 Ligand (CD40L), a factor presented by a CD4⁺ T cell to a dendritic cell that promotes dendritic cell maturation, was found to be sufficient for promoting dendritic cell induction of a CTL response. The function of CD40L can be substituted by a molecule, such as CD40 antibody, that is capable of binding to and activating CD40 in a manner similar to that of CD40L. Further, interleukin-12 (IL-12), a factor that can be produced by a mature dendritic cell, was also found to substitute for CD40L.

Therefore, it is herein described that the addition of CD40L and IL-12 can substitute for at least one function of a CD4⁺ T cell, although these factors may not effectively function to induce the production of CD8⁺ memory TL. In addition, interleukin-7 (IL-7) was found to further increase the level of CTL response. It the invention therefore comprised the addition of an effective amount of IL-7 to an incubation of a mixture of cells assembled for the generation of a CTL selective for a pathologically aberrant cell to increase the efficiency of CTL production. The use of factors such as those mentioned above will be discussed in greater detail below.

The method of the invention provides mixtures of populations of cells that can generate a CTL selective for a pathologically aberrant cell. Cell mixtures contain populations of least CD8⁺ cells, dendritic cells and pathologically aberrant cells that can be combined with other components, including CD4⁺ T cells.

Preparations of CD8⁺ L, dendritic cells, pathologically aberrant cells and CD4⁺ cells can contain many different immunological cell types. Therefore, cell mixtures assembled for inducing a CTL selective for a pathologically aberrant cell can contain cell types other than the referenced cell types.

A cell for use in the methods of the invention can be a heterogeneous population of cells, an isolated cell or a substantially isolated cell. A heterogeneous population of cells can be a naturally occurring mixture of cells that contains many immunological cell types, such as bone marrow. An isolated cell can be a cell in a fractionated population, such as a cell in a preparation of peripheral blood mononuclear cells (PBMC), or can be a population of cells that has been enriched in, or depleted of, a particular cell type. A substantially isolated cell can be a cell that is substantially free of other cell types, such as a cell that is purified.

Therefore, the cell mixtures of the invention can contain a variety of cells including referenced and non-referenced cells. The cell mixture can be assembled using a variety of cell preparations. Therefore, by altering the cell populations, it is possible to optimize a cell mixture for efficient CD8⁺ priming and CTL generation.

Cell populations contained in a CTL induction mixture can be altered in several ways, such as by preparing a cell of increased or decreased purity and by treating a particular cell type in culture to promote a particular obtainable outcome. For example, a cell can be treated with a factor that promotes cell expansion to a particular density or number, cell differentiation, specific cell lineage differentiation, apoptosis, or any desired obtainable cell condition or phenotype. Conditions that promote growth or differentiation can include protein factors or chemical reagents in the growth medium of cultured cells. These factors and reagents can be components of a crude mixture, can be isolated, or can be substantially isolated. Examples of factors include crude mixtures such as serum, naturally occurring proteins, partially purified proteins, and substantially isolated proteins.

The preparations of cell populations in a mixture can effect the efficiency of CTL priming or generation. For example, both CD8⁺ TL and dendritic cells can be prepared simultaneously in a single culture of PBMCs. Specifically, PBMCs harvested from an individual can be first depleted of CD4⁺ T cells using known methods as discussed below. One purpose of CD4⁺ T cell depletion is to promote the growth of CD8⁺ TL by preventing CD4⁺ T cells, which generally grow faster than CD8⁺ TL, from overgrowing a CD8⁺ cell population. The depleted PBMCs can then be combined with a pathologically aberrant cell. After incubating the PBMC mixture for a sufficient time that allows recognition of presented antigen by CD8⁺ TL, CTLs selective for a pathologically aberrant cell are generated.

An advantage of using one cell population for obtaining more than one cell type for assembling a mixture of cells for CTL generation is that isolation and culturing of additional cell populations is not required. In addition, by using cells from a single donor, it is known that cell types are HLA-identical, and the need to consider HLA matching of donor and recipient cell populations is therefore not required. The use of a cell mixture containing cell types from a single source is advantageous in a case in which it is difficult to obtain a source of cells, or to obtain a cell sample of sufficient amount.

Although the use of a mixture of CD8+ TL, CD4+ T cells and dendritic cells obtained from a single source provides convenience, a cell mixture can be altered to increase the efficiency of CTL generation. In order to increase the efficiency of CD8⁺ TL priming, for example, populations of cells can be individually isolated and treated in culture. Methods for isolating CD4⁺ T cells and CD8⁺ TL, dendritic cells, and pathologically aberrant cells are described in detail below. Each of the cell types of the methods of the invention can be treated in culture to enhance their particular contribution to the overall effectiveness of a mixture of cell populations to induce CD8⁺ priming or generation.

Factors that promote or augment induction of selective CTL against a target pathologically aberrant cell can be added to a culture of a mixture of cell populations or to a specific cell population cultured independently prior to assembling a cell mixture. Factors that can be useful in the methods of the invention are cytokines, growth factors, differentiation factors, proteins involved in maintenance of cell viability, and apoptosis-promoting factors.

Examples of cytokines that in addition to IL-7 can be present in an ex vivo cell culture of the methods herein described include, for example, GM-CSF and interleukins such as IL-2, and IL-12. Examples of growth factors that can be present in an ex vivo cell culture include, for example, those proteins that cause or contribute to cell growth of particular cell type employed in the methods of the invention. Specific examples of growth factors are fibroblast growth factor, platelet-derived growth factor, and transforming growth factor β. Examples of differentiation factors are those proteins that contribute to or augment the process of cell differentiation in a particular cell type of the method. A specific example of a differentiation factor is serum-derived factor. Examples of factors that contribute to the maintenance of cell viability include growth hormone and interferon alpha. Specific examples of factors used for inducing apoptosis are provided in detail below.

Factors for use in cell culture can be prepared or isolated from natural sources or produced by recombinant, methods. Specific factors, such as the cytokines IL-7 and IL-12 can be, for example, provided by a natural cell or provided in substantially purified form, such as being isolated following production by recombinant methods. Protein production by recombinant methods, detection of specific proteins in cells, and protein isolation by biochemical methods are well known in the art. Many factors can be obtained through commercial sources. Feeder cells can also supply proteins to cultured cells in membrane bound or secreted forms. Examples of cells that provide such factors are irradiated feeder cells such as irradiated cells that cannot proliferate. A specific example of a feeder cell is an irradiated monocyte that adheres to a tissue culture surface and presents or secretes factors that promote CD8+ TL priming.

The concentrations of factors used in the method of generating selective CTLs can vary depending on the particular cell types present in a mixture, the characteristics of a particular cell, the incubation time, purity of proteins and degree of isolation of cells. The characteristics of particular cell types may vary from individual to individual, resulting in different growth or differentiation rates under identical culture conditions. Concentration of factors can be adjusted to account for differences in cell behavior due to any of these differences between cell populations used to generate CTLs selective for a pathologically aberrant cell. For example, the concentration of a growth factor in a cell culture medium can be increased to promote a faster growth rate of a particular cell.

Factors can have one or more cellular functions. For example, a factor that promotes growth in a cell type may also promote differentiation in another cell type. The effect of a particular factor on a particular cell type can be determined by methods known in the art. Such methods include, for example, assay of cell number, viability, and phenotype. Specific examples of factors than can be added to the culture media of various cells including a dendritic cell and dendritic cell mixtures are described in more detail below.

For example, monocytes can be treated under conditions that promote efficient differentiation to dendritic cells. Monocytes present in or collected from PBMCs can be induced to differentiate into dendritic cells by adding GM-CSF and IL-4 for a sufficient time.

Dendritic cells can be treated in culture to increase the efficiency of uptake of antigen. Immature dendritic cells efficiently take up pathologically aberrant cells by phagocytosis, but process antigens, but present antigens less efficiently, whereas mature dendritic cells take up and process antigen less efficiently while presenting antigens more efficiently. Therefore, it is advantageous to present a pathologically aberrant cell to an immature dendritic cell prior to dendritic cell maturation. By preparing a mixture of an immature dendritic cell and a pathologically aberrant cell and allowing antigen uptake to occur prior to introducing dendritic cells to maturation factors, the efficiency of CD8+ TL priming can be increased.

Dendritic cells used in the methods of the invention can be immature or mature dendritic cells. Factors that induce dendritic cell maturation include, for example, TNF-α, LPS, and CD40L. The presence of a CD4+ T cell can also provide a dendritic cell maturation factor. Therefore, it can be advantageous to culture a dendritic cell and a pathologically aberrant cell in the absence of a CD4+ T or other maturation factor for a sufficient time in order to allow a dendritic cell to process an antigen. A dendritic cell having processed antigen can then be mixed with cells and factors that promote antigen presentation in addition to the additional cells and factors required for CTL generation. By first preparing dendritic cells that have been incubated under conditions to optimize antigen presentation, the efficiency of CD8⁺ TL priming can be increased.

The invention provides a method of inducing CD8⁺ TL ex vivo. The method comprises: contacting an apoptotic pathologically aberrant cell with a mixture having at least dendritic cells (DC), CD4⁺ T cells and CD8⁺ T lymphocytes; culturing said apoptotic pathologically aberrant cell with said mixture for sufficient time to generate CD8⁺ T lymphocytes (TL) having antigenic specificity for said pathologically aberrant cell; wherein IL-7 is added to the mixture or to the culture of apoptic pathologically aberrant cell and the mixture; culturing said CD8⁺ TL having antigenic specificity for said pathologically aberrant cell for two or more generations; and isolating CD8⁺ memory TL, said CD8⁺ memory TL being characterized as having the ability to produce CD8⁺ TL having antigenic specificity for and cytolytic activity against said pathologically aberrant cell.

An example of a cell mixture assembled for production of CTL selective for a pathologically aberrant cell is as follows. For example, one preparation of PBMCs can be fractionated to yield several cell populations. Cell populations can be isolated or substantially isolated. Specifically, monocytes can be collected from PBMCs and induced to differentiate into dendritic cells by adding GM-CSF and IL-4 for sufficient time. This immature dendritic cell population can be combined with the apoptotic pathologically aberrant cells for a sufficient period of time to allow for the uptake and processing of the pathologically aberrant cell antigens. Depletion of CD4⁺ T cells from the PBMCs can provide both a CD4⁺ T cell population and a CD4⁺-depleted PBMC population.

The resulting three cell populations can then be cultured individually. CD4⁺ T cells can be treated, for example, by radiation, to render them non-dividing, and then returned to the CD4⁺-depleted PBMCs. This PBMC population can be combined with the dendritic cell population that has been incubated with the pathologically aberrant cells, and IL-7. The pathologically aberrant cell can be, for example, a tumor cell. The mixture of cells can then be incubated for sufficient time to allow selective CTL production against the pathologically aberrant cell. The population of cells can for example, be stored, further isolated or used to treat an individual.

A method for increasing the efficiency of CD8⁺ TL priming involves enriching a population of CD4⁺ T cells with CD4⁺ T cells that recognize a specific peptide antigen associated with MHC class II molecules. In a population of CD4⁺ T cells used in the methods of the invention, only a small fraction of cells will be specific for the antigen derived from the pathologically aberrant cell and presented on the dendritic cell in the mixture of cell populations assembled to generate CTLs selective for a pathologically aberrant cell. To increase the number of antigen-specific CD4⁺ T cells in the total CD4⁺ T cell population, a method of CD4⁺ T cell enrichment can be used. For example, a CD4⁺ T cell population can be increased using an immunogen, such as tetanus toxoid. A population of CD4⁺ T cells from an individual that was previously immunized against tetanus toxoid can be obtained, cultured ex vivo, and treated with tetanus toxoid to result in an enriched population of MHC Class II restricted CD4⁺ T cells that can more effectively provide the maturation signal to immature dendritic cells. A cloned CD4⁺ T cell that is specific for an MHC class II restricted antigen can be obtained, expanded and stored for repeated use. One of skill in the art would know how to obtain a clonal cell line, expand cells and prepare cells for long-term storage.

The production of CTLs in an ex vivo cultured mixture occurs during incubation for a time period sufficient for CD8⁺ TL to recognize presented antigen and trigger commitment into CTLs. For example, the induction of selective CTLs from a cell mixture of at least dendritic cells, apoptotic pathologically aberrant cells, CD4⁺ T cells and CD8⁺ TL could occur in a time period of about 6 to 40 hours, preferably in about 20 hours. The time periods sufficient for commitment of CTL induction from a mixture of cells can be modulated by adjustment of several factors, including, for example, the amount of antigen or co-stimulatory molecules presented to a dendritic cell in a mixture of a population of cell types.

Those skilled in the art will know what factors, cellular compositions or concentrations, for example, can be used to achieve a desired incubation time. Moreover, those skilled in the art will know or can determine what factors, cellular compositions or concentrations, for example, can be adjusted to either increase or decrease the time period sufficient to prime CD8⁺ TL and commit them into maturing CTLs selective for a target pathologically aberrant cell.

For example, to determine the incubation time required for CTLs to be produced, cells can be removed from culture at various time points, for example, at 5, 15, 30, and 45 minutes, and at hourly intervals for one or more days or weeks, and tested for CTL activity or interferon gamma production after recognition of the target antigen. Pathologically aberrant cells that display the target antigen will be destroyed by selective CTLs, either through lysis of, or induction of apoptosis in, target cells. CTL activity can be measured by methods well known in the art. Methods for measuring cytotoxic activity are discussed below and in the examples in detail.

Specific factors that can further be used in the methods of the invention include IL-12, and CD40L. The concentration of IL-7 a factor required by the methods of the present invention, used in an ex vivo cultured mixture of at least dendritic cells, apoptotic pathologically aberrant cells, and CD8⁺ TL, with or without CD4⁺ T cells can be, for example, 1-30 ng/ml, 30-65 ng/ml or 65-100 ng/ml. The concentration of IL-12 in an ex vivo cultured mixture of dendritic cells, apoptotic pathologically aberrant cells, and CD8⁺ TL, for example, can be 1-30 pg/ml, 30-65 pg/ml or 65-100 pg/ml. The effective amount of CD40L presented as a cell surface protein on a natural cell or recombinant cell expressing CD40 L for use in an ex vivo cultured mixture of dendritic cells, apoptotic pathologically aberrant cells, and CD8⁺ TL can be determined by those of skill in the art. Those of skill in the art will know that the level of expression of CD40L in recombinant cells can be modulated, and that CD40L cells can be titrated into a reaction mixture to determine the effective concentration of cells to be added. Those of skill in the art will be able to extrapolate from such an effective concentration of cells to determine an effective amount of isolated or substantially isolated CD40L protein for inducing CTL selective for a pathologically aberrant cell using the methods of the invention.

For use in the methods of the invention, a cell can be irradiated. It can be advantageous to irradiate a particular cell prior to assembling the cell mixture for inducing CTL selective for a pathologically aberrant cell when growth of the particular cell during the induction period is not desired. For example, a cell such as a dendritic cell, CD4⁺ T cell, and pathologically aberrant cell can be irradiated because growth or expansion of these cell types may not be desired because increased cell numbers may not be necessary for the induction process to occur and expansion of cell types that grow at faster rates than a CD8+ T cell could provide unnecessary contamination of a generated CTL population.

As discussed previously, cells for use in the methods of the invention can be a cell mixture or an isolated cell. The preparation of CD8⁺ TL and CD4⁺ T cells, dendritic cells and pathologically aberrant cells can include isolation of a cell. A cell for use in the methods of the invention can be isolated by several methods well known in the art, such as, for example, density centrifugation, centrifugal elutriation, fluorescent-activated cell sorting, immunoaffinity cell separation, and magnetic sorting (Schindhelm and Nordon, Ex vivo Cell Therapy, (1999) Chapter 11 Academic Press, San Diego).

Density gradient centrifugation is based on the migration of cells of a particular density when centrifuged in a solution of varying density. Cells will tend to collect in a band at the zone of the gradient at which cell density and the density of the medium are equal. Examples of density gradient centrifugation methods include loading cells onto gradients prepared with reagents such as Ficoll, Ficoll-Hypaque, and Percoll.

Centrifugal elutriation is a method based on the relative differences in cell sedimentation velocity that has been well developed for separating blood cells. Fluorescent-Activated cell sorting (FACS) is a selective cell separation method by which cells labeled with fluorescent marker molecules are individually analyzed and sorted on the basis of light scatter properties and fluorescence.

Immunoaffinity cell separation is based on the interaction of cells with a substrate, such as a monoclonal antibody, lectin, or other binding domain, that is immobilized to a material with low intrinsic cell affinity. Cells selectively attach to the substrate through binding of cell surface molecules to a substrate bound to the solid-phase support. Captured cells are detached using shear stress or chemical agents that disrupt the interaction between the cell and substrate.

Magnetic sorting is a method of cell separation in which cells are labeled with an affinity substrate that contains paramagnetic or ferromagnetic material. Magnetically labeled cells can be depleted from a cell population through capture by a magnetic field, or labeled cells can be recovered by removal of a magnetic field. Dynabeads are a specific example of a magnetic cell separation medium that is commercially available.

Examples of specific methods of CD8⁺ TL isolation are negative magnetic cell selection methods, such as depletion of CD4⁺, CD14⁺, CD19⁺, or CD56+ cells, using beads coated with antibodies to cell surface proteins present on the cells to be depleted, or positive magnetic cell selection methods, such as positive selection for CD8 with magnetic beads coated with antibodies to CD8.

An example of a method for isolating dendritic cells is to collect PBMC, such as by Ficoll-Hypaque density gradient centrifugation and culture them under conditions that promote dendritic cell differentiation, as described above. Dendritic cells can be further isolated from PBMC by other known methods, such as depletion of other cell types, including CD2-, CD19- and CD56-positive cells.

Specific methods for isolating CD4⁺ T cells are, for example, negative magnetic cell separation to reduce or remove CD8⁺ TL from a cell population, and positive magnetic cell separation, to obtain an enriched population of CD4⁺ T cells.

The method for isolating a pathologically aberrant cell will be determined by the cell type and by the desired purity of the preparation. Those of skill in the art will know which methods, including those methods described above, are applicable to the isolation of a particular pathologically aberrant cell.

The populations of CD8⁺ TL and CD4⁺ T cells, dendritic cells and pathologically aberrant cells used in the method of the invention can be obtained from multiple sources. For example, cells can be obtained from an individual afflicted with a pathological condition to be treated using a CTL, or from an individual that is substantially histocompatible to the recipient individual. Such an individual might be, for example, an HLA-identical sibling, or HLA-matched relative, or HLA-matched unrelated individual. Methods for typing histocompatibility antigens are well known in the art. Using such antigen typing methods, those skilled in the art will know or can determine what level of antigen similarity is necessary for a cell to be immunologically compatible with a recipient individual. The tolerable differences between a donor cell and a recipient can vary with different tissues and are known or can be readily determined by those skilled in the art. Methods for assessing transplantation antigen compatibility are well known to those skilled in the art.

A cell for use in the methods of the invention can be obtained from a variety of tissues or organs of an individual. Those of skill in the art will know how to determine which source of a particular cell type is most suitable for the preparation of CTLs selective for a pathologically aberrant cell. It may be possible to harvest a cell from more than one source in an individual, such as more than one tissue. Those of skill in the art will be able to determine which specific source of a particular cell is preferred by considering the number of cells needed and the accessibility of the cell type in the body of an individual. For example, a particular cell type may be more abundant in one tissue compared to another tissue, or a particular tissue may be more accessible for cell harvest. An example is that a tissue such as blood is more accessible than a tissue such as bone marrow because blood cells can be removed from an individual by a relatively non-invasive procedure.

CD8⁺ TL and CD4⁺ T cells can be obtained, for example, from the blood, bone marrow, or other tissue, of an individual such as a histocompatible individual or a CTL recipient. The preferred CD8⁺ TL of the method are CD8⁺ TL that are HLA-matched with the recipient of selective CTLs. CD4⁺ T cells can be CD8⁺-autologous, or can be heterologous. A CD4⁺ T cell for use in the methods of the invention are alloreactive to a dendritic cell, or share MHC class II.

Dendritic cells, or monocytes capable of differentiating into dendritic cells, can be obtained from several tissues of an individual for use in the methods of the invention. For example, dendritic cells or monocytes can be obtained from the blood, bone marrow, or other tissue from an individual.

A pathologically aberrant cell to be used in the methods of the invention can be obtained from many sources. One source is an individual who will receive adoptive immunotherapy using CTLs generated by the methods of the invention. A pathologically aberrant cell can be a cell from a homogeneous or heterogeneous population of cells, or can be an isolated cell. Pathologically aberrant cells include cells removed from an individual, such as from a tumor or other diseased tissue or organ. The method of inducing CTL selective for pathologically aberrant cells applies to cells, homogeneous and heterogeneous cell populations, cells from natural sources such as tissues and organs, cultured cells, cultured cells that have been altered, such as by infection by a pathological agent or by transduction with nucleic acids. In addition, a wide variety of antigens, including but not limited to, cell lysate, vesicles, cell fractions or cell components, a protein or a mixture of peptides eluted from the aberrant cell can be used.

Pathologically aberrant cells of the invention can be apoptotic pathologically aberrant cells. Apoptotic pathologically aberrant cells can be present in a population of pathologically aberrant cells, can be isolated from a population of pathologically aberrant cells or can be induced in pathologically aberrant cells by several methods. Induction of apoptosis has been accomplished in a variety of ways, depending on the cell type (for review see McConkey et al. (1996) Molecular Aspects of Medicine 17:1).

Apoptosis can be triggered by the binding of cell death activators such as TNF, lymphotoxin, and Fas ligand to the extracellular domains of receptors that are integral membrane proteins. Cell death receptor activation leads to transmission of a signal to the cytoplasm resulting in activation of caspase enzymes, and a cascade of signaling events that culminate in death of a cell. Those skilled in the art will know which reagents induce apoptosis in a particular cell type or types. Examples of reagents that have been used to induce apoptosis in one or more cell lines include actinomycin D, aphidicolin, A23187, caffeine, camptothecin, cycloheximide, dexamethasone, doxorubicin, 5-fluorouracil, hydroxyurea, staurosporine, taxol, thymidine, and vinblastine (Oncogene Research Products web site).

Several methods well known in the art can be used to detect apoptotic cells. Such methods include light and electron microscopy to detect morphological changes that occur during apoptosis, flow cytometry or density gradient centrifugation to detect characteristic cell shrinkage and increased granularity, assessment of membrane integrity using dyes such as trypan blue, ethidium bromide and acridine orange, measurement of the characteristic, nonrandom DNA fragmentation using techniques including agarose gel analysis, in vitro and in situ DNA end-labeling, PCR analysis, comet assays and ELISA systems, the detection of the activity of a caspase from the caspase family of protease enzymes that are activated during apoptosis, the detection of a phosphatidylserine binding protein such as annexin V, measurement of tissue transglutaminase activity, and measurement of calcium ion flux (Promega Notes 69: technically speaking-detecting apoptosis).

Apoptotic cells can be separated from non-apoptotic cells using cell separation methods such as those described above. Therefore, an apoptotic pathologically aberrant can be present in a population of pathologically aberrant cells that naturally contains apoptotic cells, or in cells induced to contain apoptotic cells, and can be isolated from such sources for use in the methods of the invention.

The methods of the invention result in the generation of CTL selective for a pathologically aberrant cell. A CTL can be a heterogeneous population of cells, an isolated cell or substantially isolated cell. A CTL population can contain CTLs that are selective for one or more antigens on a target pathologically aberrant cell. Purification of a CTL that is specific for a pathologically aberrant cell can be performed by selecting a CTL that exhibits cytolytic activity against the target cell or antigen. Methods for selecting a CTL specific for a target and assays for measuring cytolytic activity of CTLs are well known in the art. A selected population of CTL can be purified using methods well known in the art, including the separation methods described above. A heterogeneous or isolated population of CTL produced by the methods of the invention can be characterized in vitro to determine the effectiveness of a particular CTL preparation in destroying target pathologically aberrant cells.

An example of a cytolytic activity assay is a chromium-51 (⁵¹Cr) release assay. In this method, which is described in more detail in Example II, CTL-mediated activity is determined by measuring the lysis of cultured ⁵¹Cr-labeled target cells. Another assay for measuring the function of CTLs is a target cell proliferation assay. A target cell proliferation assay measures the ability of a CTL to inhibit proliferation of target cells in vitro. Briefly, target cells are collected from an appropriate source, including for example, cell lines and primary cells isolated from an individual, and suspended in growth medium to achieve a concentration of about 10⁴ viable cells/ml for each sample to be tested. However, the amount and concentration of target cells can be adjusted according to the availability of target cells. CTLs are added to a sample at a concentration of about 10⁶ viable cells/ml, or different ratios of CTL to target cells can be can be plated to determine target cell proliferation at differing CTL concentrations. The ratios of effector to target cells can be between about 500:1 to 0.1:1. Cells are typically incubated for about 1 to 24 hours.

It can be desirable to have control over the lifespan of a CTL generated using the methods of the invention. One method for controlling the viability of a cell is to introduce a suicide gene that metabolizes a compound taken up by a cell to produce a toxin that kills the cell. For example, introduction of a thymidine kinase gene into a CTL can render the CTL susceptible to killing by addition of ganciclovir. Using this method, a CTL expressing thymidine kinase can be eliminated if the CTL or the associated target-selective cytolytic activity are not needed or desired. The use of the thymidine kinase gene and the suicide substrate ganciclovir is well known in the art. Methods of transducing a vector containing a thymidine kinase gene into a cell are well known in the art.

CD8⁺ memory CTLs selective for a pathologically aberrant cell can also be generated using the methods of the invention. A CD8⁺ memory CTL selective for a pathologically aberrant cell can be obtained by culturing CTL having selective cytolytic activity toward a pathologically aberrant for two or more generations. A CD8+ memory CTL has the capability to respond to antigen more efficiently than a naïve CD8+ TL, and can be re-stimulated with antigen in an ex vivo culture. A CD8⁺ memory CTL can be distinguished from a CTL in an ex vivo culture because a CD8⁺ memory CTL have the ability to undergo multiple restimulations, typically at least fire restimulations. Isolation of a memory CD8⁺ TL selective for a pathologically aberrant cell can be achieved by methods well known in the art, including those methods described above.

A CTL that is selective for a target antigen of a pathologically aberrant cell is useful for the same therapeutic applications as a CTL selective for a pathologically aberrant cell because the result of target antigen recognition is lysis of a cell by the CTL selective for the target antigen. Therefore, CTLs selective for a target antigen generated in ex vivo culture can be useful for adoptive immunotherapy.

As discussed previously, the use of CTLs specific for target antigens that are peptide antigens have not been predictably successful. This approach could be improved by the inclusion of a factor that could increase the probability of producing a CTL selective for a target antigen. The methods of the invention provide the advantage of increased efficiency of CTL production by utilizing IL-7 in a mixture of cells in ex vivo culture. The use of IL-7, as provided in the methods of the invention, increases the efficiency of producing CTL response toward target cells and thus increases the probability that a CTL selective for a target antigen will be produced.

The invention provides a method of inducing CD8⁺ TL selective for one or more target antigens ex vivo. The method consists of contacting said one or more target antigens with a mixture having at least dendritic cells (DC), CD4⁺ T cells, CD8⁺ TL and IL-7, and culturing said one or more target antigens with said mixture for sufficient time to generate CD8⁺ cytolytic T lymphocytes (CTL) having selective immune reactivity toward said one or more target antigens.

Herein described is also a method of identifying an antigen selective for a pathologically aberrant cell. CTLs that have been generated against the pathologically aberrant cell or its products, using the methods of the invention, can be used to identify the antigen that they recognize. This can be achieved using procedures that are known to those skilled in the art. One such method consists in purifying the Class I MHC from the pathologically aberrant cell and releasing the peptides associated with it. The peptide mixture is then separated in fractions and analyzed to determine the fraction containing the antigenic peptide. The peptide sequence is then determined using available techniques (such as the techniques of Argonex, Charlottesville, VA which are commercially available; and Hunt et al., Science (1992) 255:5049). The limiting material for utilizing these techniques is the antigen-specific CD8⁺ TL, which are provided using the methods of the present invention.

Another described method consists of: (a) treating a pathologically aberrant cell with a mutagenizing agent to produce a mutant population of pathologically aberrant cells; (b) contacting said mutant population of pathologically aberrant cells with a CTL selective for said pathologically aberrant cell to identify a mutant pathologically aberrant cell that has lost reactivity with said CTL; (c) introducing an expressible population of nucleic acids coding for a pathologically aberrant cell polypeptides into said mutant cell to produce a population of mutant cells expressing said polypeptides, and (d) identifying a mutant cell expressing a pathologically aberrant cell polypeptide that restores reactivity with said CTL reactive for said pathologically aberrant cell.

A population of CTLs selective for a pathologically aberrant cell or target antigen can be used to identify an antigen selective for the pathologically aberrant cell. Populations of CTLs used for identifying selective antigens can exhibit, for example, poly-specific or monospecific reactivity toward the pathologically aberrant cell. The method for identifying an antigen of a pathologically aberrant cell or unknown target antigen involves obtaining a population of target pathologically aberrant cells that has been mutagenized so that an antigen recognized by a CTL selective for a pathologically aberrant cell is no longer present, introducing a cDNA library into the antigen-deficient pathologically aberrant cells, and selecting a pathologically aberrant cell that expresses a cDNA which restores the ability of a CTL to recognize the pathologically aberrant cell. A cDNA is then rescued from the cell and sequenced to determine the identity of a target antigen.

Mutagenesis of a population of pathologically aberrant cells can be performed using methods well known in the art. For example, cells can be mutated by methods such as irradiation or treatment with chemical mutagens, such as ethyl methanesulfonate. A mutated cell that, still retains Class I and the antigen processing machinery, and is not recognized by a CTL selective for the pathologically aberrant cell can be identified because it will not be lysed by a CTL. The introduction of a cDNA library into the antigen-deleted cells can be performed by methods well known in the art, such as transfection using chemical reagents and electroporation. A transduced cell that can be recognized by a CTL contains a cDNA encoding a protein that is recognized by the CTL. The identification of an antigen-restored cell can be performed by screening a transduced cell population using the functional activity of the CTLs selective for the pathologically aberrant cell. Rescue and sequencing of the cDNA is then performed using methods well known in the art.

Further described is a method of identifying an antigen selective for a pathologically aberrant cell. The method consists of: (a) contacting one or more antigens suspected of being selective for a pathologically aberrant cell with a CTL selective for a pathologically aberrant cell expressing said one or more antigens, and (b) determining the immunoreactivity of said CTL selective for a pathologically aberrant cell expressing said one or more antigens toward said one or more antigens, wherein a CTL having selective immunoreactivity for said one or more antigens characterizes said one or more immunoreactive antigens as being selective for said pathologically aberrant cell.

A population of CTLs selective for a pathologically aberrant cell or for one or more target antigens generated using the methods of the invention can also be used to identify or corroborate the selectivity of one or more target antigens suspected of being selective for a pathological aberrant cell and therefore, associated with a particular disease or condition. Briefly, the method involves screening one or more target antigens with a CTL population or CTL clone selective for a pathologically aberrant cell expressing the suspect one or more antigens. Selective immunoreactivity of the CTLs towards the suspect antigen indicates or confirms the selectivity of the antigen for the pathologically aberrant cell. Similarly, the suspect antigens can be screened by first expressing the antigens in a host cell and then determining the cytolytic activity of the CTLs towards the cells expressing the suspect antigens. Selective cytolytic activity indicates or confirms the selectivity of the one or more antigens for the pathologically aberrant cell. Cells that express one or more suspect antigens can be, for example, naturally occurring cells or cells modified with expressible nucleic acids encoding the suspect target antigen. Methods for modifying cells with nucleic acids and identifying the expression of polypeptides in cells are well known in the art.

An antigen identified using the above method can be used as a target for the development of therapeutic agents. For example, libraries of small molecule compounds and peptides can be screened to identify molecules that specifically bind to an antigen, or specific therapeutic antibodies can be raised to the antigen. An antigen identified using this method can also be used for the development of diagnostic methods.

CTL selective for a pathologically aberrant cell generated using the methods of the invention are applicable for the treatment of human conditions or diseases that involve the pathologically aberrant cell. CTL that are determined to have selective cytolytic activity in vitro or in situ functional assays such as those described above are likely to have selective cytolytic activity in an individual because a CTL will recognize the target cell or antigen in a heterogeneous population. Therefore, provided in the invention are methods for treating an individual with a CTL, including a memory CTL, selective for a pathologically aberrant cell or target antigen, generated using the methods of the invention.

Described is also a method of treating a patient having a disease mediated by a pathologically aberrant cell. The method consists of administering an effective amount of a CD8⁺ CTL having selective cytolytic activity toward said pathologically aberrant cell; said CD8⁺ CTL having selective cytolytic activity toward said pathologically aberrant cell being produced using the methods of the invention.

Further described is a method of treating a patient having a disease mediated by a pathologically aberrant non-B-cell leukemia cell. The method consists of administering an effective amount of a CD8⁺ CTL having selective cytolytic activity toward said pathologically aberrant non-B-cell leukemia cell, said CD8⁺ CTL having selective cytolytic activity toward said pathologically aberrant non-B-cell leukemia cell being produced by the methods of the invention.

Further described is a method of treating a patient having a disease mediated by a pathologically aberrant cell. The method consists of administering an effective amount of a CD8⁺ CTL having selective immune reactivity toward one or more target antigens associated with said pathologically aberrant cell, said CD8⁺ CTL having selective immune reactivity being produced by the methods of the invention.

As previously described, there are many types of pathologically aberrant cells, all of which are distinguished from normal cells. Pathologically aberrant cells can be treated with a CTL because a CTL selective for a pathologically aberrant cell can selectively recognize and destroy that cell within a population of normal cells. Therefore, the use of CTLs that selectively target pathologically aberrant cells is applicable to treating individuals in which the destruction of such cells can reduce the severity of disease or slow the rate of disease progression.

Those of skill in the art will know how to determine if treatment with CTLs selective for a pathologically aberrant cell is beneficial for a particular pathological condition, and will know how to determine the presence of a pathologically aberrant cell in an individual having a pathological condition. Those of skill will be able to determine if pathologically aberrant cells of a disease can be eliminated without causing unwanted effects in an individual. For example, it is advantageous to remove a tumor cell that serves no beneficial function in the body. However, eliminating a cell that is diseased but nevertheless provides a function in the body, such as an essential cell of an organ, is not generally desired. However, there are certain non-vital organs and cells from vital or non-vital organs that can be removed without affecting the survival of the individual, including for example, but not limited to, the prostate, ovary, breast, thyroid, thymus, selected cell types of vital and non-vital organs, or B cells that synthesize IgE. In this case, CTLs that recognize a self-antigen that is shared by a diseased cell and a normal cell or tissue or organ can be used.

The amount of CTLs effective for treating a pathological condition is an amount required to affect a decrease in target cell population or slow the rate of increase in a target cell population. The therapeutically effective dose will depend, for example, on the pathological condition characterized by the pathologically aberrant cell to be treated, the route and form of administration, the weight and condition of the individual, and previous or concurrent therapies. For example, pathologically aberrant cells that are localized to one region in the body of an individual may be effectively treated by injection of CTLs at that particular site.

The injected dose can vary depending on the size of the population of pathologically aberrant cells such that a relatively large population of cells can require a relatively large dose of CTLs. The weight of an individual can effect the dosage of CTL delivered by a systemic route, such as, for example, intravenous infusion. For example, for treating an individual with hematopoietic cancer in which pathologically aberrant cells are circulating in the blood, the effective dose would be determined, at least in part, by the body weight of the individual so that that the concentration of administered CTL is approximately the same for low body weight individuals, such as children, and higher body weight individuals, such as adults.

The appropriate effective dose for a particular application of the methods can be determined by those skilled in the art, using the guidance provided herein. For example, the amount can be extrapolated from in vitro or in vivo assays as described previously. One skilled in the art will recognize that the condition of the patient can be monitored throughout the course of therapy and that the amount of CTL preparation that is administered can be adjusted accordingly.

An effective amount of CTLs selective for a pathologically aberrant cell to administer can be determined by those of skill in the art, who will know how to perform tests to determine the efficacy of a dose of CTLs used for treating a particular disease or condition. Those of skill in the art can also determine whether CTLs selective for a pathologically aberrant cell can be most effectively administered as a single dose or multiple doses.

A preparation of CTLs can be delivered systemically, such as by intravenous infusion, or can be administered locally at a site of the pathological condition, such as by injection. Appropriate sites for administration of a CTL preparation are known, or can be determined, by those skilled in the art depending on the clinical indications of the individual being treated.

A CTL selective for a pathologically aberrant cell can be administered as a suspension together with a pharmaceutically acceptable medium. Such a pharmaceutically acceptable medium can be, for example, a buffered saline solution. Pharmaceutically acceptable mediums can be sterile or substantially free from contaminating particles and organisms. Those of skill in the art will know or be able to determine pharmaceutically acceptable media for CTL to be used in various modes of administration.

A pathological condition can be treated with a CTL, a memory CTL, or a combination of a CTL and memory CTL that are selective for a pathologically aberrant cell. A CTL and memory CTL can be administered simultaneously or on separate occasions. Administration of a memory CTL can provide the advantage of long term immunity against a pathologically aberrant cell and can thus prevent relapse of disease. Those of skill in the art will know or can determine which type of CTL to use for effective treatment of a particular disease or condition.

The described methods of treating a pathological condition characterized by aberrant cell growth additionally can be practiced in conjunction with other therapies. For example, for treating cancer, the methods of the invention can be practiced prior to, during, or subsequent to conventional cancer treatments such as surgery, chemotherapy, including administration of cytokines and growth factors, radiation or other methods known in the art. Similarly, for treating pathological conditions, including infectious disease, the methods of the invention can be practiced prior to, during, or subsequent to conventional treatments, such as antibiotic administration, against infectious agents or other methods known in the art. Treatment of pathological conditions of autoimmune disorders also can be accomplished by combining the selective CTL cell elimination methods of the invention with conventional treatments for the particular autoimmune diseases. Conventional treatments include, for example, chemotherapy, steroid therapy, insulin and other growth factor and cytokine therapy, passive immunity, inhibitors of T cell receptor binding and T cell receptor vaccination. It may be advantageous to treat an individual receiving CTL selective for a pathologically aberrant cell with an immunostimulant. Those of skill in the art will be able to determine the particular immunostimulant and appropriate time and mode of administration of an immunostimulant.

The methods herein described can be administered in conjunction with these or other methods known in the art and at various times prior, during or subsequent to initiation of conventional treatments. For a description of treatments for pathological conditions characterized by aberrant cell growth see, for example, The Merck Manual, Sixteenth Ed, (Berkow, R., Editor) Rahway, NJ, 1992.

Similarly, other cell therapy methods well know in the art can additionally be employed in conjunction with selective CTLs generated by the methods of the invention. Such other methods include, for example, cell replacement therapy for the regeneration or reconstitution of tissues and cellular components thereof, and cell therapy using genetically modified cells for the production of a therapeutic protein or macromolecule. Specific examples of cell replacement therapy include hematopoietic stem and progenitor cell therapy, which can be used, for example, to reconstitute ablated bone marrow cells of cancer patients, and neuronal stem and progenitor cell therapy for the treatment of Parkinson's disease. Cell therapy for production of a therapeutic protein includes, for example, the transplantation of a variety of cell types and progenitors thereof genetically modified to produce, for example, insulin, other cytokines, growth factors, and enzymes. Such genetic cell therapies are applicable, for example, to the treatment of diabetes, cancer. Various other examples of cell replacement and genetic cell therapy are well known in the art and are similarly applicable for use in conjunction with the selective CTLs produced by the methods of the invention.

The administration of CTLs selective for a pathologically aberrant cell simultaneous with or delivered in alternative administrations with the conventional therapy, including multiple administrations. Simultaneous administration can be, for example, together in the same formulation or in different formulations delivered at about the same time or immediately in sequence. Alternating administrations can be, for example, delivering a CTL preparation and conventional therapeutic treatment in temporally separate administrations. As described previously, the temporally separate administrations of selective CTLs and conventional therapy can similarly use different modes of delivery and routes.

Also herein described is the preparation of mature dendritic cells. The mature dendritic cells produced by the methods of this invention could then be used for vaccination, including administration in combination with the adoptive transfer of CTL. This method comprises preparing immature dendritic cells, as herein described, pulsing them with antigen (such as apoptotic cells, vesicles, cell lysates, cell fractions or components, proteins or peptides) and a T helper epitope and incubating these dendritic cells with helper T cells specific for the helper epitope associated with the Class II MHC of the dendritic cell.

The following examples are intended to illustrate but not limit the present invention.

### EXAMPLE 1

### CONDITIONS FOR ACTIVATION OF CD8⁺ CYTOTOXIC ACTIVITY BY DENDRITIC CELLS

This example shows that stimulation of dendritic cells (DC) with either CD40L or IL-12 induces CTL activity in CD8⁺ TL in vitro, and that IL-7 augments this response.

To determine the optimal conditions for in vitro induction of human CTL, HLA-A2 DC were used to activate purified CD8⁺ T lymphocytes (CD8⁺ TL). The response to a class I MHC antigen was investigated because the high precursor frequency of CD8⁺ TL specific for class I MHC antigens allows for the detection of a CTL response after only one round of in vitro stimulation. The preparation of HLA-A2 DC and CD8⁺ TL are described below.

CD8⁺ TL were isolated from PBMC of HLA-A2-negative healthy blood donors. CD8⁺ positive cells were recovered after positive selection with Dynabeads and Detachabead (Dynal, Lake Success, NY) and stored frozen prior to use. After thawing, cells were further depleted of CD4⁺, CD14⁺, CD19⁺, and CD56⁺ with Dynabeads. Fluorescence-activated cell sorter (FACS)-analysis showed that this cell population was >99% CD8⁺. DC were generated from peripheral blood monocytes essentially as described by F. Sallusto and A. Lanzavecchia, J. Exp. Med. (1992) 176:1693-1702. HLA-A2-positive PBMC from healthy blood donors were isolated by Ficoll-Hypaque density gradient centrifugation and suspended at a concentration of 4 x 10⁶/ml in RPMI-FCS medium. Of this suspension, 15-ml aliquots were plated in 150-mm tissue culture dishes. After 90 minutes at 37°C, non-adherent cells were discarded, and plates were washed five times with PBS and subsequently incubated at 37°C for 30 minutes in the presence of PBS containing 2% FCS and 2% EDTA. Adherent cells were detached by pipetting followed by scraping. The cell suspension was then further depleted of CD2-, CD19-, and CD56⁺-positive cells with Dynabeads (Dynal AS, Oslo, Norway) according to the manufacturer's instructions. This cell population (<0.5% CD3⁺ and >90% CD14⁺) was plated in six well-plates (1.5 x 10⁶ cells/well) in RPMI-FCS in the presence of 500 U/ml human recombinant IL-4 (Genzyme, Boston, MA) and 800 U/ml human recombinant granulocyte-monocyte colony stimulating factor GM-CSF (Pharmingen, San Diego, CA).

Induction of HLA-A-2 specific CTL was performed on cells cultured in RPMI 1640 supplemented with 2 mM L-glutamine, 50 µg/ml gentamycin 1% non-essential amino acids, 1mM sodium pyruvate, and 5% pooled human serum (RPMI-HS). DC (10⁴ cells/well) and CD8⁺ cells (10⁵ cells/well), prepared as described above, were cultured in 96 well flat-bottomed plates in a final volume of 200 µl. Cells were incubated for six days at 37°C prior to CTL activity assay.

The cytotoxic activity of CTL was quantitatively measured by ⁵¹CR-release assay using JY (A2⁺) and 221 (A2⁻) ⁵¹Cr-labeled target cells. The assay was performed by splitting the 96 well culture plate into two sets of replicate plates (96-well U-bottomed). For each set, one plate contained 15 µl and the other plate contained 75 µl of the original culture. One set received ⁵¹Cr-labeled 221 target cells (10⁴ cells/well) while the other set received ⁵¹Cr-labeled JY target cells (10⁴ cells/well). In order to decrease any antigen non-specific cytotoxic activity due to NK activity, all the wells received 2 x 10⁵ K562 cells. After six hours at 37°C, the ⁵¹Cr content in 100 µl of supernatant was measured, and the percent specific lysis was calculated according to the formula: percent lysis = 100 x (experimental release - spontaneous release) / (maximum release - spontaneous release). For proper comparison of the efficiency of the different conditions tested, the data are expressed as lytic units (LU) 30/10⁶ input cells. One LU is defined as the number of input cells required to achieve 30% lysis of 10⁴ target cells in a six hour assay. Specific CTL activity is obtained by subtracting the LU obtained from 221 cells from the LU obtained from JY cells. The period of exposure or incubation of CTLs with target cells can vary depending on whether quantitative or qualitative results are desired. Generally, the period of exposure is between about 30 minutes and six hours, preferably the period is between about two to five hours and more preferably the period is about three to four hours. K562, a human natural killer (NK)-sensitive cell line; 3A4-721.221, an Epstein-Barr virus (EBV)-transformed cell line that has been mutagenized and selected to be class I negative, and JY, an EBV-transformed HLA-A2 homozygous cell line, were grown in RPMI-1640 media (Gibco, Grand Island, NJ) containing 10% fetal calf serum (FCS; Sigma, St. Louis, MO), 4 mM L-glutamine, 1% nonessential amino acids, 1 mM sodium pyruvate, 5 x 10⁻⁵ M 2-mercaptoethanol, and 50 µg/ml gentamycin (all from Gibco, Grand Island, NJ) (RPMI-FCS).

To determine if different DC maturation signals effected CD8⁺ activation, DC were challenged with lipopolysaccharide (LPS), tumor necrosis factor-α (TNF-α), or CD40L, and the ability of treated DC to induce CTL activity in purified CD8⁺ cells was determined by the ⁵¹Cr-release as described. DC prepared as described above were treated with either TNF-α, (20 ng/ml), LPS (20 ng/ml), or no additive (immature DC). Following a 40 hour incubation at 37°C, DC were collected and plated. DC were challenged with CD40L at the time of culture with the CD8⁺ cells by adding irradiated (25,000 rads) CD40L-transfected cells (Cella et al., J. Exp. Med. 184: 747-752 (1996)) to the immature DC.

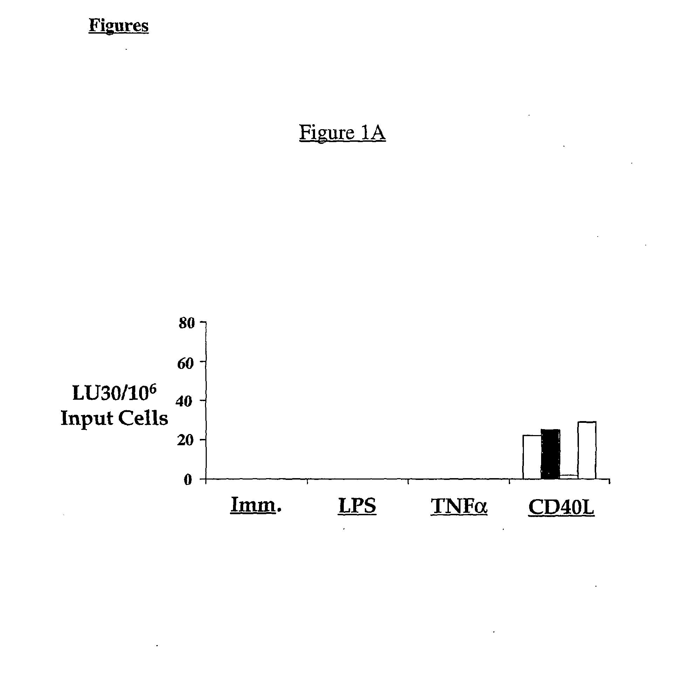

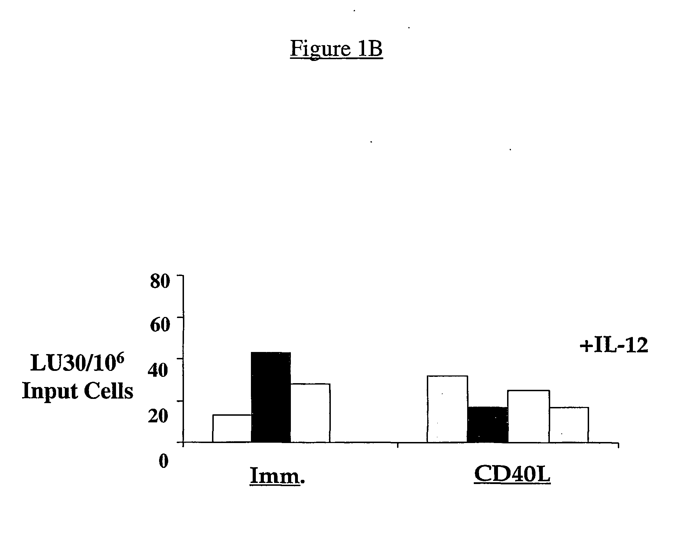

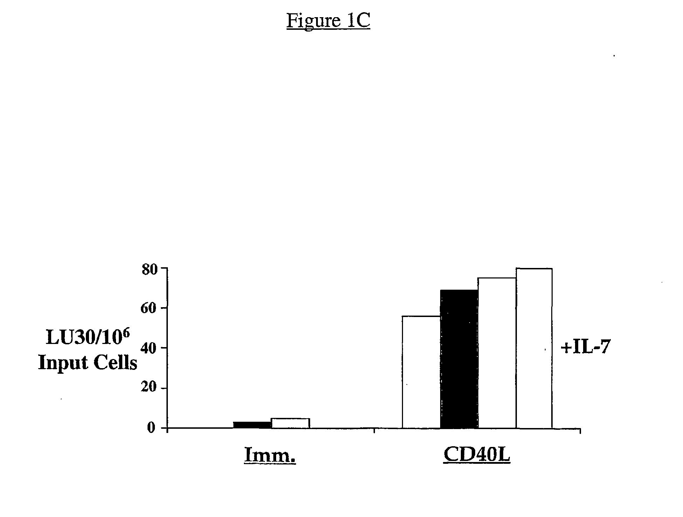

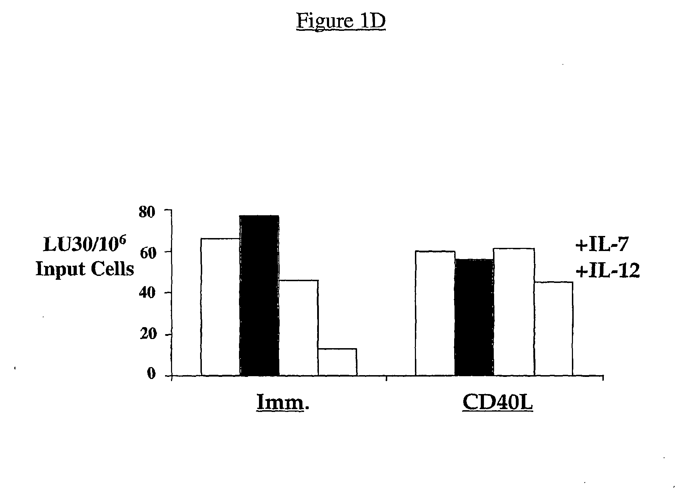

The results from four representative experiments are shown in Figure 1. Cytotoxic activity induced by immature DC (imm-DC), LPS treated DC (LPS-DC), TNF-α-treated DC (TNF-DC), or CD40L-treated DC (CD40L-DC) indicated that only CD40L treated DC were capable of inducing cytotoxic activity from purified CD8⁺ T cells (Figure 1A). CTL activity was detected in three of the four tested CD8⁺ cell populations.

To determine if the differences observed between DC treated with LPS, TNF-α, or CD40L in inducing CTL activity of CD8⁺ T cells were due to differences in antigen processing and presentation capacity of monocytes, immature DC, and mature DC, the expression levels of class I molecules, TAP transporters, PA28 (an immunoproteasome regulator), and immunoproteasomes were examined by flow cytometry (FACScan, Benton Dickenson, Milano, Italy) and immunoprecipitation with appropriate antibodies. After treatment with GM-CSF and IL-4 as described above, the resulting immature DC exhibited increased expression of class I molecules, TAP transporters, PA28, and immunoproteasomes by four to five fold as compared to untreated monocytes. The level of expression of class I molecules was further increased by two fold upon treatment with LPS, CD40L, or TNF-α. The expression levels of class I molecules in mature DC were not effected by the various treatments, indicating that differences observed between DC treated with LPS, TNFα, or CD40L in inducing CTL activity of CD8⁺ T cells are not the result of differences in antigen processing and presentation capacity.

The cytokine IL-12 is known to have an important role in CTL development and function (Trinchieri, Ann. Rev. Immunol. (1995) 13:251-76). A study was performed to determine if induction of cytotoxicity by CD40L-DC could be substituted by IL-12 and/or increased by other lymphokines (Figure 1). Cytotoxic activity induced by imm-DC or CD40L-DC in the presence of IL-12 (Figure 1B), IL-7 (Figure 1C), or IL-12 plus IL-7 (Figure 1D) was measured. Addition of IL-4 or IL-6 had no effect on cytotoxic activity. The addition of IL-12 to immature DC was able to substitute for CD40L in two of the four purified CD8⁺ cell populations. IL-7 had no effect on immature DC but enhanced the cytotoxic activity induced by CD40L-treated DC and IL-12-treated immature DC by approximately three -fold. Furthermore, in the presence of IL-7, cytotoxic activity was detected in all four CD8⁺ populations. Thus, the treatment that resulted in the most consistent induction of cytotoxic response for all CD8⁺ donors, and in all systems tested, was the combination of CD40L-DC and IL-7. The tested systems included the CTL response against the super antigen TSST, against the A2-restriced matrix peptide derived from the influenza A virus, and against the A2-restriced tyrosinase peptide.

### EXAMPLE 2

### INDUCTION OF LB-SPECIFIC CD8⁺ CTL LINES

This example shows that CD8⁺ CTL activity is induced in response to specific leukemicblasts (LB) and demonstrates the development of CD8⁺ CTL cell lines.

The observation that CD40L and IL-12 in combination with IL-7 effectively promoted DC-induced CTL activity of CD8⁺ TL provided the basis for developing of a procedure for inducing LB-specific CTL. Due to the low amount of blood available from BMT donors and recipients, the previously described procedures were modified as follows. Briefly, DC were prepared by adding IL-4 and GM-CSF directly to adherent PBMC isolated from BMT donors immediately after non-adherent cells were removed. After a seven day culture period, DC expressed a pattern of activation antigens similar to a phenotype described for resting DC activated by mechanical manipulation (Gallucci et al., Nature Medicine (1999) 5:1249-1255). CD8⁺ TL were obtained by negative depletion of CD4⁺ cells.

The studies described in this Example and subsequent Examples included four pediatric acute myeloid leukemia (AML) patients, given allogenic bone marrow transplants (BMT), and their donors. None of the BMT recipients experienced relapse. The follow-up ranged from 23 to 28 months after transplant. Three patients (MR, GA, and PM) received BMT from an HLA-identical sibling, whereas patient EV received BMT from an HLA-matched unrelated donor. BMT recipients were evaluated for LB-specific cytotoxic activity for six months after transplant, when immunosuppressive therapy had ceased. The institutional review board of the Department of Pediatrics Science, IRCCS Policlinico San Matteo, approved the protocol.

In an initial study, LB-specific CTL were induced by priming CD8⁺ TL (CD4⁺<1%) using LB plus DC together with IL-7 and IL-12. It was observed that CD8⁺ TL from two BMT donors (MR-don and GA-don) contained LB-specific CTL after a second stimulation, but that these cultures were difficult to maintain due to their low propensity to expand (data not shown).

To overcome the problem of lack of expansion of LB-specific CTL lines, CD4⁺-enriched irradiated autologous cells were added to a CD8⁺ TL priming incubation. This approach was used to generate leukemia-specific CTL lines from CD8⁺ TL derived from either peripheral blood or bone marrow of four BMT donors (MR-don, GA-don, EV-don, and PM-don). The medium used was RPMI-HS supplemented with 10 ng/ml IL-7 and 10 pg/ml IL-12. CD8⁺ cells (0.5 to 1 x 10⁶ cells/ml) were added to 48 well-plates and co-cultured with irradiated (20,000 rads) BMT recipient LB (5 x 10⁵ cells/ml), irradiated (3,000 rads) CD8⁺-autologous CD4⁺ lymphocytes (3 to 5 x 10⁵ cells/ml) and CD8⁺-autologous DC (2 x 10⁵ cells/ml) in a final volume of 1 ml.

After seven days, cultures were re-stimulated with irradiated (20,000 rads) BMT recipient LB (5 x 10⁵ cells/ml) and adherent irradiated (3000 rads) feeder cells (Vitiello et al., J. Clin. Invest. 95: 351-349 (1995)). Two days later, 25 U/ml of recombinant interleukin-2 (rIL-2) was added to the cultures. The same protocol was used for each successive round of stimulation. LB were prepared from heparinized bone marrow aspirates (>90% LB) from patients at the time of their diagnosis.

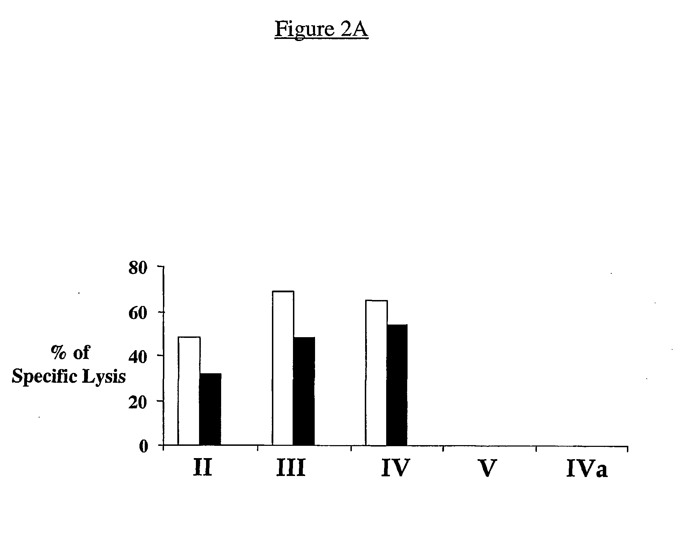

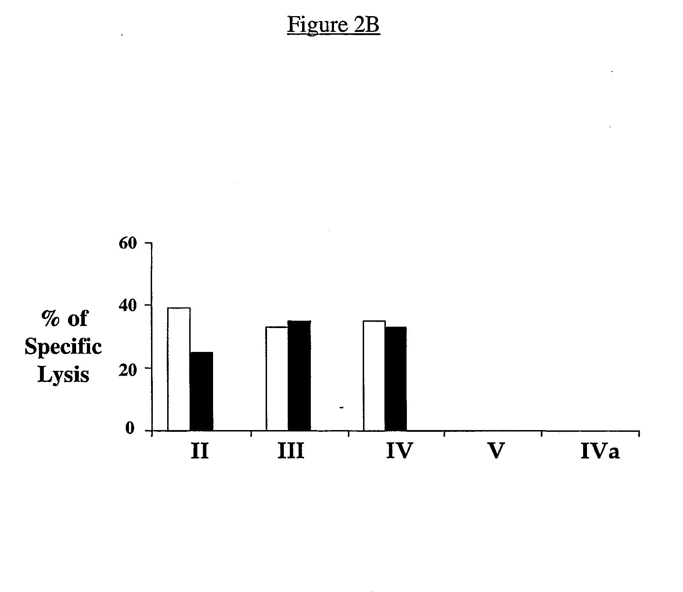

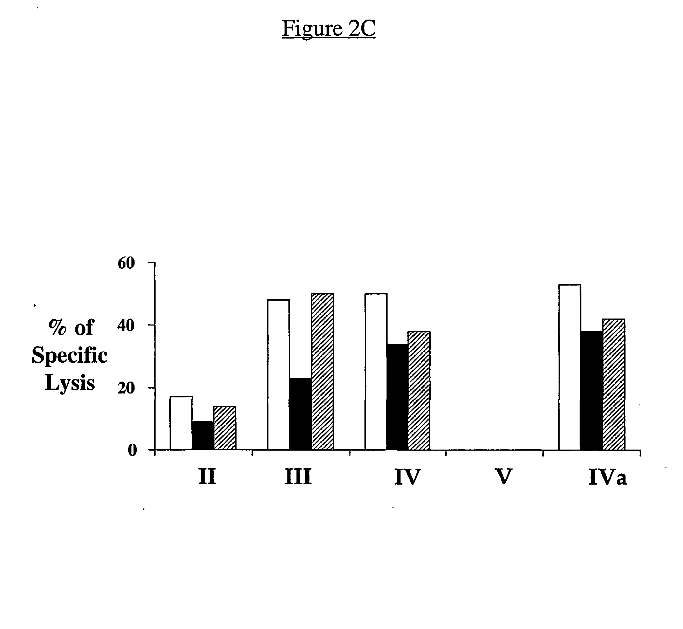

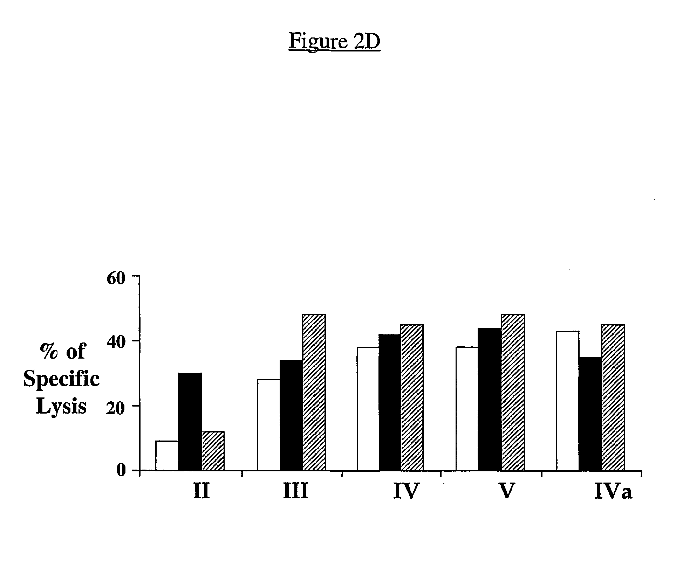

Cultures were tested for cytolytic activity seven days after each subsequent re-stimulation against recipient LB at an effector to target (E:T) ratio of 5:1 (Figure 2). Cytolytic activity of LB-specific CTL lines derived from PBMC of BMT recipient (□), BMC of BMT donor (■) and from PBMC of BMT donor ( 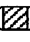 ) was determined.
Cell culture stimulations performed at seven day intervals are represented by II, III, IV, and V in Figure 2. Cytolytic activity of thawed LB-directed CTL, cryopreserved after the fourth re-stimulation, is represented as IVa. Figure 2 (A-D), refer to MR, GA, EV, PM donor/recipient pairs, respectively.

This study revealed that cytolytic activity was not observed after the primary stimulation. However, after the second stimulation (14 to 16 days following the initial culturing), LB-specific cytotoxicity was observed in all cultures tested and was maintained or augmented after further re-stimulation. The same kinetics and magnitude of CTL activation were observed when CD8⁺ cells were obtained from PBMC of the four BMT recipients (MR, GA, EV, and PM) six months after transplant, when hematopoietic and immune systems were of donor origin. The cytotoxic capacity of LB-specific CTL lines was not affected by cryopreservation.

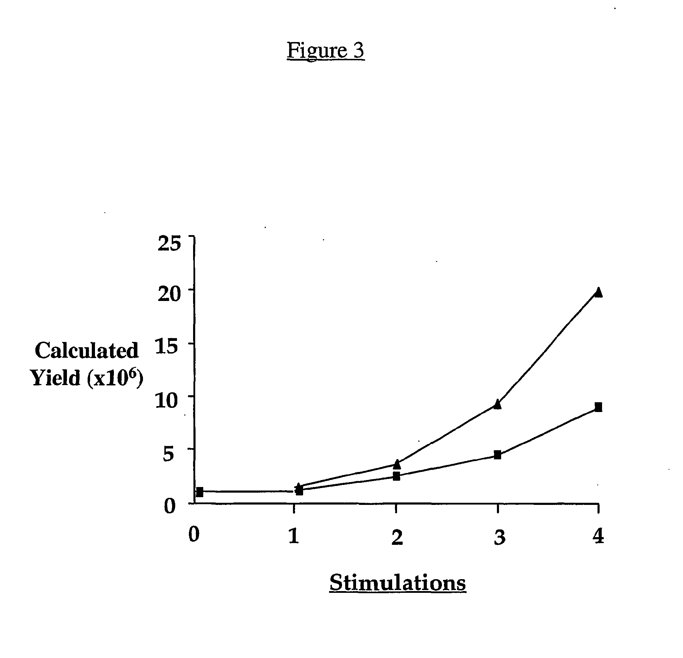

The ability of LB-directed CTL to expand in culture in the presence of DC, target cells, IL-12 and IL-7, as described above, was examined. Figure 3 shows representative results of cell expansion of LB-directed CTL derived from BMT recipient EV (▲) or BMT donor EV (■) PBMC. Data represent a theoretical calculation based on the expansion rates of the CTL. The expansion rates of the CTL were calculated as follows: expansion rate = total number of the cells after re-stimulation/number of re-stimulated cells. The initial number of CD8⁺-enriched responder cells was 10⁶. After repeated stimulation CTL demonstrated an incremental increase in cytotoxicity and a variable but continuous expansion of the absolute number of cultured cells. These cells were expanded in the range of nine to twenty times compared to cells seeded at the beginning of the cultures.

In view of the potential use of LB-specific CTL lines for adoptive immunotherapy, the cell lines were tested against non-leukemic cells obtained from patients before transplant as an in vitro control for the graft versus host reaction (GVHR). The autologous target cells used were Bone marrow recipient cells (BMRC), and Mitogen-stimulated T-cells (T-PHA). Non-leukemic BMRC were obtained before the BMT procedure and after demonstration of complete haematological remission. T-PHA cell lines were established for each patient by stimulating cryopreserved pre-transplant PBMC with PHA and serial passaged in RPMI-FCS supplemented and 100 U/ml of rIL-2 (Cetus, Emeryville, CA). The majority of cultures showed either no or low reactivity (<10% lysis) at the highest effector to target ratio (E:T ratio) tested (up to 40:1) against non-leukemic BMR cells or T-PHA.

To exclude reactivity of the CTL lines against recipient antigens, the frequency of CTL precursors (CTLp) directed to the pre-transplant, non-leukemic recipient cells of patient EV was evaluated by limiting dilution assay (LDA) as previously described (Montagna, et al., J. Clin. Immunol. (1996) 16:107-114). The patient's donor was an HLA-matched unrelated donor. Of the four donor-recipient pairs, this pair was at highest risk of GVHD since the other three pairs were HLA-identical siblings. Analysis of CTLp frequency was performed for: (I) PBMC of the donor before any in vitro activation, (ii) CD8⁺ effector cells obtained from the donor, recovered after the fourth stimulation with LB cells, and (iii) CD8⁺ effector cells obtained from the recipient six months after BMT, recovered after the fourth stimulation with LB cells. The results in Table II demonstrate that, after four rounds of in vitro LB-stimulation, the frequency of CTLp against recipient pre-transplant non-leukemic cells did not increase compared to the frequency obtained from donor PBMC before stimulation with the LB cells.

**Table 2.**

| **CTLP FREQUENCY TOWARDS RECIPIENT'S SELF-ANTIGENS DOES NOT CHANGE AFTER IN VITRO STIMULATION WITH LB AND DC** | |
|---|---|
| **Source** | **CTLp Frequency** |
| PBMC EV don | 1/381,000 |
| CTL* EV pt | 1/325,000 |
| CTL* EV don | 1/317,000 |

| | |
|---|---|
| * LB-specific CTL lines obtained after 4 stimulations and tested on pre-transplant PHA blasts of BMT recipient. | |

### EXAMPLE 3

### CHARACTERIZATION OF LB-SPECIFIC CTL LINES

This example shows the phenotypic analysis of LB-specific CTL lines.

Phenotypic analysis of LB-specific CTL cell lines was performed at the time of each cytotoxicity assay. Representative results obtained from primary cultures and after the fourth stimulation are reported in Table III. In primary cultures, when LB-directed cytotoxic activity was undetectable, CD3⁺ and/or CD8⁺ lymphocytes ranged from 32% to 79%, whereas a considerable proportion of CD56⁺ lymphocytes (ranging from 20% to 66%) was present. After the fourth stimulation, the great majority of effector cells were CD3⁺ and CD8⁺ lymphocytes, while the proportion of CD56⁺ cells decreased to less than 20%. Interestingly, CD4⁺ cells also increased from less than 1% before stimulation to around 10% by the fourth round of stimulation.

**TABLE 3.**

| **SURFACE PHENOTYPE OF LB-SPECIFIC CTL LINES** | | | | |
|---|---|---|---|---|
| | | | | |

| **CTL LINES BMT-RECIPIENTS** | **EFFECTOR PRIMARY CELLS AFTER STIMULATION** | | **EFFECTOR CELLS AFTER 4 STIMULATIONS** | |
|---|---|---|---|---|
| | % | (range) | % | (range) |
| CD3 | 48 | (42-57) | 94 | (93-96) |
| CD8 | 45 | (32-55) | 82 | (73-88) |
| CD4 | 5 | (4-6) | 8 | (7-10) |
| CD56 | 50 | (36-66) | 14 | (11-20) |
| γδ | 10 | (5-14) | 30 | (13-65) |

| **CTL LINES* BMT-DONORS** | **EFFECTOR CELLS AFTER PRIMARY STIMULATION** | | **EFFECTOR CELLS AFTER 4 STIMULATIONS** | |
|---|---|---|---|---|
| CD3 | 66 | (58-79) | 92 | (87-97) |
| CD8 | 50 | (41-62) | 84 | (80-88) |
| CD4 | 6 | (3-10) | 11 | (8-15) |
| CD56 | 28 | (20-30) | 9 | (6-15) |
| Γδ | 8 | (5-16) | 21 | (3-52) |

| | | | | |
|---|---|---|---|---|
| *Obtained from PBMC. | | | | |

### EXAMPLE 4

### LB-SPECIFIC CTL LINES ARE CD8⁺ CLASS I RESTRICTED

This example shows that cytolytic activity of CTL was restricted to CD8⁺ cells and HLA class I antigen. To determine which T-cell populations were mediating the lysis of the leukemia cells, the cytotoxic assays were performed in the presence of blocking antibodies. Target cells were incubated with anti-HLA class I or class II monoclonal antibodies (mAb) (25 µg/ml), and effector cells were incubated with mAb specific for CD4⁺ and CD8⁺ for 30 minutes at 4°C. The same concentration of each mAb was added to cultures after about four hours from the beginning of cytotoxicity assay.

After three in vitro stimulations, CTL lines, obtained from PBMC of BMT recipients (PBMC rec) and from bone marrow cells (BM don) or PBMC (PBMC don) of BMT donors, were tested for CTL activity, at an E/T ratio of 10:1, against LB target cells in the presence of media only (□), and-HLA class I(■), anti-HLA class II ( 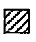 ), anti-CD8⁺ ( 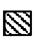 ) and anti-CD4⁺ ( 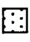 ) mAbs (Figure 4). Representative experiments of LB specific CTL lines derived from EV (Figure 4A) and PM (Figure 4B) donor/recipient pairs are reported.

In all cases, LB-reactive lysis was inhibited by anti-HLA class I and anti- CD8⁺ mAb but not by anti-HLA class II and anti-CD4⁺ mAb, indicating that CD8⁺ cells were responsible for the cytolysis. After repeated stimulation, CD4⁺ cells contained in LB-specific CTL lines ranged between 5% and 12%. Thus, depletion experiments of CD4⁺ cells were performed before cytotoxicity assays to exclude the involvement of this subpopulation in mediating cytolytic activity. These results demonstrated that LB-directed cytotoxic activity was unaffected by depletion of CD4⁺ effector cells.

### EXAMPLE 5

### CTL-MEDIATED LB LYSIS IS PERFORIN DEPENDENT

This example shows that LB-directed cytotoxicity is inhibited by concanamycin A and strontium chloride:

Cytolytic activity of a CTL refers to the secretion of the contents of secretory granules into the immediate vicinity of the target cell to which it attaches. The granules contain perforins, various lysosomal enzymes, and calcium binding proteins that destroy the target cell. To identify the mechanism responsible for target lysis in LB-directed cytotoxic activity, the role of the granule exocytosis pathway was analyzed by using either concanamycin A (CMA), an inhibitor of vacuolar type H⁺-ATPase, or strontium chloride (SrCl₂), which causes degranulation and release of granule contents from effector cells.

Effector cells were treated with concanamycin A (CMA) (Sigma, Milano, Italy) at concentrations of 1, 10, and 100 nM/L for two hours, or with SrCl₂ (Sigma) at concentrations of 5, 25, and 50 mM/L for 18 hours, washed twice, and then incubated with ⁵¹Cr-labeled recipient LB for eight hours at E:T ratios of 20:1, 10:1, and 5:1. Shown in Figure 5 are the results of studies performed with an E: T ratio of 5:1, demonstrating the effect of CMA (Figure 5A) and SrCl₂ (Figure 5B) on cytotoxic activity displayed from LB-directed CTL obtained from the PBMC of recipient PM (■), the BMC of donor PM (▲), and the PBMC of donor PM (•) after three stimulations.

Thus, LB-directed cytotoxicity was inhibited by treatment with either CMA or SrCl₂ (mean inhibition = 87%, Figure 5). Because these reagents selectively block perforin-based target lysis it was concluded that target cell lysis is perforin-dependent.

## Claims

1. An *ex vivo* method of inducing CD8⁺ T cells selective for a pathologically aberrant cell, comprising: contacting an apoptotic pathologically aberrant cell with a mixture having at least dendritic cells (DC), CD4⁺ T cells and CD8⁺ T lymphocytes; culturing said apoptotic pathologically aberrant cell with said mixture for sufficient time to generate CD8⁺ T lymphocytes (TL) having antigenic specificity for said pathologically aberrant cell; wherein IL-7 is added to the mixture or to the culture of apoptic pathologically aberrant cell and the mixture; culturing said CD8⁺ TL having antigenic specificity for said pathologically aberrant cell for two or more generations; and isolating CD8⁺ memory TL, said CD8⁺ memory TL being **characterized** as having the ability to produce CD8⁺ TL having antigenic specificity for and cytolytic activity against said pathologically aberrant cell.

2. The method of Claim 1, wherein said pathologically aberrant cell further comprises a cell selected from a group consisting of a tumor cell, a cell infected with a pathological agent, a vesicle, a cell from a non-vital organ, a B cell, cell lysates, cell fractions, cell components, and cells producing a substance that mediates a disease or condition.

3. The method of Claim 1, wherein said CD8⁺ TL further comprise naive CD8⁺ TL.

4. The method of Claim 1, wherein said DCs further comprise substantially isolated DCs.

5. The method of Claim 1, wherein said CD4⁺ T cells further comprise substantially isolated CD4⁺ T cells.

6. The method of Claim 1, wherein said CD8⁺ TL further comprise substantially isolated CD8⁺ TL.

7. The method of Claim 1, wherein said pathologically aberrant cell is a non-B-cell leukemia cell.

8. The method of Claim 7, wherein said pathologically aberrant non-B-cell leukemia cell is a B-cell lymphoma cell.

9. The method of Claim 7, wherein said pathologically aberrant non-B-cell leukemia cell is a T-cell leukemia cell.

10. An *ex vivo* method of inducing CD8⁺ T lymphocytes (TL) selective for a pathologically aberrant cell, comprising:
a) contacting said pathologically aberrant cell with a first mixture having at least dendritic cells (DC) and isolated CD4⁺ T cells for sufficient time to produce DCs presenting pathologically aberrant cell antigen;
b) adding CD8⁺ TL to produce a second mixture;
c) culturing said second mixture for sufficient time to generate CD8⁺ TL having antigenic specificity for said pathologically aberrant cell;
d) culturing said CD8⁺ TL having antigenic specificity for said pathologically aberrant cell for two or more generations; and
e) isolating CD8⁺ memory TL, said CD8⁺ memory TL being **characterized** as having the ability to produce CD8⁺ TL having antigenic specificity for said pathologically aberrant cell;
wherein IL-7 is added in at least one of step (a), step (b), or step (c).

11. The method of Claim 10, wherein said pathologically aberrant cell further comprises a tumor cell, a vesicle, a cell lysate, a cell fraction, a cell component, a cell from a vital or non-vital organ, a B cell, cells producing a substance that mediates a disease or condition, or a cell infected with a pathological agent.

12. The method of Claim 10, wherein said CD8⁺ TL further comprise naive CD8⁺ TL.

## Patentansprüche

1. *Ex vivo* Verfahren zur Induzierung von CD8⁺ T Zellen, die für eine pathologisch aberrante Zelle selektiv sind, umfassend in Kontakt bringen einer apoptotischen pathologisch aberranten Zelle mit einem Gemisch, das mindestens dendritische Zellen (DC), CD4⁺ T Zellen und CD8⁺ T Lymphozyten aufweist; Kultivieren der apoptotischen pathologisch aberranten Zelle mit dem Gemisch für eine ausreichende Zeit, um CD8⁺ T-Lymphozyten (TL) zu erzeugen, die eine antigenische Spezifität für die pathologisch aberrante Zelle aufweisen; wobei IL-7 zu dem Gemisch oder zu der Kultur der apoptotischen pathologisch aberranten Zelle und dem Gemisch hinzugefügt wird; Kultivieren der CD8⁺ TL, die die antigenische Spezifität für die pathologisch aberrante Zelle aufweisen, für zwei oder mehr Generationen; und Isolieren von CD8⁺ Gedächtnis-TL, wobei die CD8⁺ Gedächtnis-TL **dadurch gekennzeichnet sind, daß** sie die Fähigkeit aufweisen, CD8⁺-TL zu produzieren, die eine antigenische Spezifität für und zytolytische Aktivität gegen die pathologisch aberrante Zelle aufweisen.

2. Verfahren nach Anspruch 1, wobei die pathologisch aberrante Zelle weiter eine Zelle umfaßt, ausgewählt aus der Gruppe bestehend aus einer Tumorzelle, einer mit einem pathologischen Agens infizierten Zelle, einem Vesikel, einer Zelle aus einem nicht lebenswichtigen Organ, einer B Zelle, Zelllysaten, Zellfraktionen, Zellkomponenten und Zellen, die eine Substanz herstellen, die eine Erkrankung oder einen Zustand vermittelt.

3. Verfahren nach Anspruch 1, wobei die CD8⁺-TL weiter naive CD8⁺-TL umfassen.

4. Verfahren nach Anspruch 1, wobei die DCs weiter im wesentlichen isolierte DCs umfassen.

5. Verfahren nach Anspruch 1, wobei die CD4⁺-T Zellen weiter im wesentlichen isolierte CD4⁺-T Zellen umfassen.

6. Verfahren nach Anspruch 1, wobei die CD8⁺-TL weiter im wesentlichen isolierte CD8⁺-TL umfassen.

7. Verfahren nach Anspruch 1, wobei die pathologisch aberrante Zelle eine nicht-B-Zell-Leukämiezelle ist.

8. Verfahren nach Anspruch 7, wobei die pathologisch aberrante nicht-B-Zell-Leukämiezelle eine B-Zell Lymphomzelle ist.

9. Verfahren nach Anspruch 7, wobei die pathologisch aberrante nicht-B-Zell-Leukämiezelle eine T-Zell Leukämiezelle ist.

10. *Ex vivo* Verfahren zur Induzierung von CD8⁺ T Lymphozyten (TL), die für eine pathologisch aberrante Zelle selektiv sind, umfassend:
a) in Kontakt bringen der pathologisch aberranten Zelle mit einem ersten Gemisch, das mindestens dendritische Zellen (DC) und isolierte CD4⁺ T Zellen aufweist, für eine ausreichende Zeit, um DCs herzustellen, die pathologisch aberrante Zelle-Antigen präsentieren;
b) Hinzufügen von CD8⁺ TL, um ein zweites Gemisch herzustellen;
c) Kultivieren des zweiten Gemischs für eine ausreichende Zeit, um CD8⁺ T-Lymphozyten (TL) zu erzeugen, die eine antigenische Spezifität für die pathologisch aberrante Zelle aufweisen;
d) Kultivieren der CD8⁺ TL, die eine antigenische Spezifität für die pathologisch aberrante Zelle aufweisen, für zwei oder mehr Generationen; und
e) Isolieren von CD8⁺ Gedächtnis-TL, wobei die CD8⁺ Gedächtnis-TL **dadurch gekennzeichnet sind, daß** sie die Fähigkeit aufweisen CD8⁺-TL zu produzieren, die eine antigenische Spezifität für die pathologisch aberrante Zelle aufweisen;
wobei IL-7 in mindestens einem von Schritt (a), Schritt (b) oder Schritt (c) hinzugefügt wird.

11. Verfahren nach Anspruch 10, wobei die pathologisch aberrante Zelle weiter eine Tumorzelle, ein Vesikel, ein Zelllysat, eine Zellfraktion, eine Zellkomponente, eine Zelle aus einem lebenswichtigen oder nicht lebenswichtigen Organ, eine B Zelle, Zellen, die eine Substanz herstellen, die eine Erkrankung oder einen Zustand vermittelt, oder eine Zelle, die mit einem pathologischen Agens infiziert ist, umfaßt.

12. Verfahren nach Anspruch 10, wobei die CD8⁺-TL weiter naive CD8⁺-TL umfassen.

## Revendications

1. Procédé *ex vivo* d'induction de lymphocytes T CD8⁺ sélectifs pour une cellule pathologiquement aberrante, comprenant les étapes consistant à: mettre en contact une cellule pathologiquement aberrante avec un mélange ayant au moins des cellules dendritiques (CD), des lymphocytes T CD4⁺ et des lymphocytes T CD8⁺; cultiver ladite cellule pathologiquement aberrante en cours d'apoptose avec ledit mélange pendant une durée suffisante pour générer des lymphocytes T CD8⁺ (LT) ayant une spécificité antigénique pour ladite cellule pathologiquement aberrante; dans lequel de l'IL-7 est ajouté au mélange ou à la culture de cellule pathologiquement aberrante en cours d'apoptose et au mélange; cultiver lesdits LT CD8⁺ ayant une spécificité antigénique pour ladite cellule pathologiquement aberrante pour deux générations ou plus; et isoler les LT mémoire CD8⁺, lesdits LT mémoire CD8⁺ étant **caractérisés** comme ayant la capacité à produire des LT CD8⁺ ayant une spécificité antigénique pour, ladite cellule pathologiquement aberrante et une activité cytolytique contre elle.

2. Procédé selon la revendication 1, dans lequel ladite cellule pathologiquement aberrante comprend de plus une cellule choisie dans un groupe consistant en une cellule tumorale, une cellule infectée avec un agent pathologique, une vésicule, une cellule provenant d'un organe non vital, un lymphocyte B, des lysats cellulaires, des fractions cellulaires, des composants cellulaires et des cellules produisant une substance médiatrice d'une maladie ou d'un état.

3. Procédé selon la revendication 1, dans lequel lesdits LT CD8⁺ comprennent de plus des LT CD8⁺ naïfs.

4. Procédé selon la revendication 1, dans lequel les CD comprennent en outre des CD sensiblement isolées.

5. Procédé selon la revendication 1, dans lequel lesdits lymphocytes T CD4⁺ comprennent de plus des lymphocytes T CD4⁺ sensiblement isolés.

6. Procédé selon la revendication 1, dans lequel lesdits LT CD8⁺ comprennent de plus des LT CD8⁺ sensiblement isolés.

7. Procédé selon la revendication 1, dans lequel ladite cellule pathologiquement aberrante est une cellule de leucémie à cellules non B.

8. Procédé selon la revendication 7, dans lequel ladite cellule de leucémie à cellules non B pathologiquement aberrante est une cellule de lymphome à cellules B.

9. Procédé selon la revendication 7, dans lequel ladite cellule de leucémie à cellules non B pathologiquement aberrante est une cellule de leucémie à cellules T.

10. Procédé *ex vivo* d'induction de lymphocytes T (LT) CD8⁺ sélectifs pour une cellule pathologiquement aberrante, comprenant les étapes consistant à:
a) mettre en contact ladite cellule pathologiquement aberrante avec un premier mélange ayant au moins des cellules dendritiques (CD) et des lymphocytes T CD4⁺ isolés pendant une durée suffisante pour produire des CD présentant un antigène de cellule pathologiquement aberrante;
b) ajouter des LT CD8⁺ pour produire un second mélange;
c) cultiver ledit second mélange pendant une durée suffisante pour générer des LT CD8⁺ ayant une spécificité antigénique pour ladite cellule pathologiquement aberrante;
d) cultiver lesdits LT CD8⁺ ayant une spécificité antigénique pour ladite cellule pathologiquement aberrante pendant deux générations ou plus; et
e) isoler les LT mémoire CD8⁺, lesdits LT mémoire CD8⁺ étant **caractérisés** comme ayant la capacité à produire des LT CD8⁺ ayant une spécificité antigénique pour ladite cellule pathologiquement aberrante;
dans lequel de l'IL-7 est ajouté à au moins l'une de l'étape (a), de l'étape (b) ou de l'étape (c).

11. Procédé selon la revendication 10, dans lequel ladite cellule pathologiquement aberrante comprend en outre une cellule tumorale, une vésicule, un lysat cellulaire, une fraction cellulaire, un composant cellulaire, une cellule dérivée d'un organe vital ou non vital, une cellule B, des cellules produisant une substance médiatrice d'une maladie ou d'un état, ou une cellule infectée par un agent pathologique.

12. Procédé selon la revendication 10, dans lequel lesdits LT CD8⁺ comprennent des LT CD8⁺ naïfs.
